# EUROPEAN PATENT APPLICATION

(11) **EP 4 742 264 A1**
(43) Date of publication of application: **13.05.2026**
(21) Application number: 24838751.6
(22) Date of filing: 05.07.2024
(51) Int. Cl.: G16H 30/20

(54) **METHOD AND APPARATUS FOR PRESENTING ORAL HEALTH EXAMINATION INFORMATION, AND DEVICE AND STORAGE MEDIUM**

(30) Priority: 07.07.2023 CN 202310838383; 07.07.2023 CN 202310838151; 28.09.2023 CN 202311296139; 28.09.2023 CN 202311289263; 22.12.2023 CN 202311789536
(71) Applicant: Shining 3D Tech Co., Ltd., Hangzhou, Zhejiang 311258 (CN)
(72) Inventor: ZHAO, Xiaobo, Hangzhou, Zhejiang 311258 (CN); NI, Kaijia, Hangzhou, Zhejiang 311258 (CN); YAN, Kexin, Hangzhou, Zhejiang 311258 (CN); MA, Chao, Hangzhou, Zhejiang 311258 (CN); ZHANG, Huiquan, Hangzhou, Zhejiang 311258 (CN); CHEN, Xiaojun, Hangzhou, Zhejiang 311258 (CN); LU, Yan, Hangzhou, Zhejiang 311258 (CN); JIANG, Tengfei, Hangzhou, Zhejiang 311258 (CN)
(74) Representative: Metida
(86) International application number: PCT/CN2024/104069
(87) International publication number: WO 2025/011498

(57) **Abstract**

The present application provides a method, an apparatus, a device, and a storage medium for displaying oral health examination information. The method includes: displaying a plurality of tooth images on a visualization interface, wherein a tooth image represents a two-dimensional image of a three-dimensional model from one of perspectives of the three-dimensional model; in response to an operation instruction of a user for a specified tooth image, rotating the three-dimensional model to a perspective corresponding to the specified tooth image and displaying the three-dimensional model on the visualization interface; wherein the specified tooth image includes a tooth image displaying a suspected lesion area, and the perspective corresponding to the specified tooth image is determined by pose parameters of the three-dimensional model bound to the tooth image. Through the above method, a patient can directly see a position of the suspected lesion area on the three-dimensional model from the visualization interface, thereby allowing the patient to intuitively understand the oral health examination information through the visualization interface.

## Description

The present application claims priority to Chinese Patent Application No. 202310838151.3, filed with the China National Intellectual Property Administration on July 7, 2023, entitled "METHOD AND APPARATUS FOR GENERATING ORAL EXAMINATION REPORT, COMPUTER DEVICE"; Chinese Patent Application No. 202310838383.9, filed with the China National Intellectual Property Administration on July 7, 2023, entitled "METHOD AND APPARATUS FOR DISPLAYING ORAL EXAMINATION INFORMATION, COMPUTER DEVICE"; Chinese Patent Application No. 202311296139.0, filed with the China National Intellectual Property Administration on September 28, 2023, entitled "METHOD, APPARATUS, DEVICE, AND MEDIUM FOR DISPLAYING ORAL HEALTH EXAMINATION INFORMATION"; Chinese Patent Application No. 202311289263.4, filed with the China National Intellectual Property Administration on September 28, 2023, entitled "METHOD AND APPARATUS FOR GENERATING ORAL HEALTH EXAMINATION REPORT, COMPUTER DEVICE"; and Chinese Patent Application No. 202311789536.1, filed with the China National Intellectual Property Administration on December 22, 2023, entitled "SCREENSHOT MANAGEMENT METHOD, APPARATUS, COMPUTER DEVICE AND MEDIUM". The entire contents of all the aforementioned applications are incorporated herein by reference.

### TECHNICAL FIELD

The present application relates to the field of oral medicine technology, and in particular, to a method for displaying oral health examination information, an apparatus, a device, and a storage medium.

### BACKGROUND

In some scenarios, when users communicate based on two-dimensional images, one party may lack corresponding professional knowledge, making it difficult to understand the content explaining the two-dimensional images, resulting in low communication efficiency. For example, in medical scenarios, doctors typically communicate with patients using paper-based medical images. However, due to patients' lack of certain professional knowledge, they have difficulty understanding the doctor's explanation of the medical images. Doctors cannot well understand the patient's needs and expectations, and it is difficult to help patients understand treatment plans and expected outcomes.

Doctor-patient communication can help doctors better understand the patient's needs and expectations, and can also help patients better understand treatment plans and expected outcomes.

In related technologies, when doctors communicate the condition with patients after performing an oral examination, due to patients' lack of certain professional knowledge, they have difficulty understanding oral health examination information through the doctor's verbal description.

Furthermore, generating an oral health examination report after performing an oral health examination for a patient is an important medical service provided by medical institutions. The oral health examination report usually records the patient's basic information and condition information. Doctors can use it to track and manage the patient's condition, better understand the patient's condition and treatment effectiveness, in order to further develop more effective treatment plans.

In related technologies, an oral health examination report is generated based on a fixed oral health examination report template. The names of various oral diseases displayed on this oral health examination report are all generic names, which cannot meet the customized needs of medical institutions in different regions.

### SUMMARY

In view of the above, the present application provides a method for displaying oral health examination information, an apparatus, a device, and a storage medium to address deficiencies in related technologies.

A first aspect of the present application provides a method for displaying oral health examination information. The method includes: displaying a plurality of tooth images on a visualization interface, the tooth image representing a two-dimensional image of a three-dimensional model from one of perspectives of the three-dimensional model; in response to an operation instruction of a user for a specified tooth image, rotating the three-dimensional model to a perspective corresponding to the specified tooth image and displaying the three-dimensional model on the visualization interface; wherein the specified tooth image comprises a tooth image displaying a suspected lesion area, and the perspective corresponding to the specified tooth image is determined by pose parameters of the three-dimensional model bound to the tooth image.

A first aspect of the present application provides a method for displaying oral health examination information. The method further includes: in response to a template acquisition instruction carrying a template ID; acquiring a target oral health examination report template from a server based on the template ID, the server being configured to provide an oral health examination report template set, the oral health examination report template set comprising a plurality of oral health examination report templates generated for a same oral disease, the oral health examination report template defining a candidate name for an oral disease, the candidate name being a generic name or a custom name; acquiring oral health examination information, the oral health examination information being obtained based on a result of recognizing the three-dimensional model by a pre-trained oral detection model, and/or based on a result of recognizing the tooth image by a user, the oral health examination information comprising at least one suspected lesion area and an oral disease name corresponding to each suspected lesion area; generating an oral health examination report on the target oral health examination report template based on the oral health examination information, to display the oral disease name as the candidate name defined in the target oral health examination report template.

A first aspect of the present application provides a method for displaying oral health examination information. The method further includes: in response to a screenshot instruction issued by a user, capturing an image of a specified area in the visualization interface, the specified area comprising at least a portion of the three-dimensional model; binding the image of the specified area with pose parameters of the three-dimensional model, and saving the image of the specified area into an image list; the image list comprises a plurality of images, and the image of the specified area represents a two-dimensional image of the three-dimensional model from one of perspectives of the three-dimensional model.

A second aspect of the present application provides a computer device. The computer device includes a processor and a machine-readable storage medium. The machine-readable storage medium stores machine-executable instructions executable by the processor. The processor executes the machine-executable instructions to implement: displaying a plurality of tooth images on a visualization interface, the tooth image representing a two-dimensional image of a three-dimensional model from one of perspectives of the three-dimensional model; in response to an operation instruction of a user for a specified tooth image, rotating the three-dimensional model to a perspective corresponding to the specified tooth image and displaying the three-dimensional model on the visualization interface; wherein the specified tooth image comprises a tooth image displaying a suspected lesion area, and the perspective corresponding to the specified tooth image is determined by pose parameters of the three-dimensional model bound to the tooth image.

A second aspect of the present application provides a computer device. The computer device includes a processor and a machine-readable storage medium. The machine-readable storage medium stores machine-executable instructions executable by the processor. The processor executes the machine-executable instructions to further implement: in response to a template acquisition instruction carrying a template ID; acquiring a target oral health examination report template from a server based on the template ID, the server being configured to provide an oral health examination report template set, the oral health examination report template set comprising a plurality of oral health examination report templates generated for a same oral disease, the oral health examination report template defining a candidate name for an oral disease, the candidate name being a generic name or a custom name; acquiring oral health examination information, the oral health examination information being obtained based on a result of recognizing the three-dimensional model by a pre-trained oral detection model, and/or based on a result of recognizing the tooth image by a user, the oral health examination information comprising at least one suspected lesion area and an oral disease name corresponding to each suspected lesion area; generating an oral health examination report on the target oral health examination report template based on the oral health examination information, to display the oral disease name as the candidate name defined in the target oral health examination report template.

A second aspect of the present application provides a computer device. The computer device includes a processor and a machine-readable storage medium. The machine-readable storage medium stores machine-executable instructions executable by the processor. The processor executes the machine-executable instructions to further implement: in response to a screenshot instruction issued by a user, capturing an image of a specified area in the visualization interface, the specified area comprising at least a portion of the three-dimensional model; binding the image of the specified area with pose parameters of the three-dimensional model, and saving the image of the specified area into an image list; the image list comprises a plurality of images, and the image of the specified area represents a two-dimensional image of the three-dimensional model from one of perspectives of the three-dimensional model.

A third aspect of the present application provides a computer-readable storage medium. The medium stores a computer program. The computer program, when executed by a processor, implements a method including: displaying a plurality of tooth images on a visualization interface, the tooth image representing a two-dimensional image of a three-dimensional model from one of perspectives of the three-dimensional model; in response to an operation instruction of a user for a specified tooth image, rotating the three-dimensional model to a perspective corresponding to the specified tooth image and displaying the three-dimensional model on the visualization interface; wherein the specified tooth image comprises a tooth image displaying a suspected lesion area, and the perspective corresponding to the specified tooth image is determined by pose parameters of the three-dimensional model bound to the tooth image.

A third aspect of the present application provides a computer-readable storage medium. The medium stores a computer program. The computer program, when executed by a processor, further implements a method including: in response to a template acquisition instruction carrying a template ID; acquiring a target oral health examination report template from a server based on the template ID, the server being configured to provide an oral health examination report template set, the oral health examination report template set comprising a plurality of oral health examination report templates generated for a same oral disease, the oral health examination report template defining a candidate name for an oral disease, the candidate name being a generic name or a custom name; acquiring oral health examination information, the oral health examination information being obtained based on a result of recognizing the three-dimensional model by a pre-trained oral detection model, and/or based on a result of recognizing the tooth image by a user, the oral health examination information comprising at least one suspected lesion area and an oral disease name corresponding to each suspected lesion area; generating an oral health examination report on the target oral health examination report template based on the oral health examination information, to display the oral disease name as the candidate name defined in the target oral health examination report template.

A third aspect of the present application provides a computer-readable storage medium. The medium stores a computer program. The computer program, when executed by a processor, further implements a method including: in response to a screenshot instruction issued by a user, capturing an image of a specified area in the visualization interface, the specified area comprising at least a portion of the three-dimensional model; binding the image of the specified area with pose parameters of the three-dimensional model, and saving the image of the specified area into an image list; the image list comprises a plurality of images, and the image of the specified area represents a two-dimensional image of the three-dimensional model from one of perspectives of the three-dimensional model.

A fourth aspect of the present application provides an apparatus for displaying oral health examination information. The apparatus includes: an image display module, configured to display a plurality of tooth images on a visualization interface, wherein the tooth image represents a two-dimensional image of a three-dimensional model from one of perspectives of the three-dimensional model; a model rotation module, configured to, in response to an operation instruction of the user for a specified tooth image, rotate the three-dimensional model to a perspective corresponding to the specified tooth image and display the three-dimensional model on the visualization interface; wherein the specified tooth image comprises a tooth image displaying a suspected lesion area, and the perspective corresponding to the specified tooth image is determined by pose parameters of the three-dimensional model bound to the tooth image.

A fourth aspect of the present application provides an apparatus for displaying oral health examination information. The apparatus further includes: a template acquisition module, configured to, in response to a template acquisition instruction carrying a template ID, acquire a target oral health examination report template from a server based on the template ID, the server being configured to provide an oral health examination report template set, the oral health examination report template set including a plurality of oral health examination report templates generated for a same oral disease, the oral health examination report template defining a candidate name for an oral disease, the candidate name being a generic name or a custom name; an information acquisition module, configured to acquire oral health examination information, the oral health examination information being obtained based on a result of recognizing the three-dimensional model by a pre-trained oral detection model, and/or, based on a result of recognizing the tooth image by a user, the oral health examination information including at least one suspected lesion area and an oral disease name corresponding to each suspected lesion area; a report generation module, configured to generate an oral health examination report on the target oral health examination report template based on the oral health examination information, to display the oral disease name as the candidate name defined in the target oral health examination report template.

A fourth aspect of the present application provides an apparatus for displaying oral health examination information. The apparatus further includes: a model screenshot module, configured to, in response to a screenshot instruction issued by a user, capture an image of a specified area in the visualization interface, the specified area comprising at least a portion of the three-dimensional model; an image storage module, configured to bind the image of the specified area with pose parameters of the three-dimensional model, and save the image of the specified area into an image list; the image list comprises a plurality of images, and the image of the specified area represents a two-dimensional image of the three-dimensional model from one of perspectives of the three-dimensional model.

As can be seen from the above technical solutions, the technical solution of the present application, by responding to an operation instruction of a user for a tooth image displaying a suspected lesion area, displays on the visualization interface the three-dimensional model rotated to the perspective corresponding to the tooth image, enabling the patient to directly see the position of the suspected lesion area on the three-dimensional model from the visualization interface, thereby allowing the patient to intuitively understand the oral health examination information through the visualization interface.

The technical solution of the present application, by, when generating an examination report, acquiring a target oral health examination report template from an oral health examination report template set provided by a server, and generating an oral health examination report on the target oral health examination report template based on the acquired oral health examination information, because the oral health examination report template set includes a plurality of oral health examination report templates that define different custom names for the same disease, enables the generated oral health examination report to display custom names for oral diseases, thereby meeting the reading habits of medical institutions in different regions, improving work and communication efficiency, and satisfying the customized needs of medical institutions in different regions.

The technical solution of the present application, by binding the captured image including at least a portion of the three-dimensional model with the pose parameters of the three-dimensional model and saving it into an image list, can then respond to an operation instruction of the user for a specified image in the image list and display on the visualization interface the three-dimensional model rotated to the perspective corresponding to the specified image, enabling users to intuitively communicate based on the three-dimensional model displayed on the visualization interface, making it easier to understand highly professional content and improving communication efficiency.

It should be understood that the above general description and the following detailed description are exemplary and explanatory only and do not limit the present application.

### BRIEF DESCRIPTION OF THE DRAWINGS

The accompanying drawings, which are incorporated in and constitute a part of this specification, illustrate embodiments consistent with the present application and together with the description serve to explain the principles of the present application.
FIG. 1 is a flowchart illustrating a method for displaying oral health examination information according to an embodiment of the present application;
FIG. 2 is a schematic diagram of a visualization interface according to an embodiment of the present application;
FIG. 3 is another schematic diagram of a visualization interface according to an embodiment of the present application;
FIG. 4 is another schematic diagram of a visualization interface according to an embodiment of the present application;
FIG. 5 is a schematic diagram of a three-dimensional tooth model displaying labels according to an embodiment of the present application;
FIG. 6 is a schematic diagram of a three-dimensional tooth model in a closed state according to an embodiment of the present application;
FIG. 7 is a schematic diagram of a three-dimensional tooth model in an open state according to an embodiment of the present application;
FIG. 8 is a schematic diagram of a three-dimensional tooth model displaying tooth areas according to an embodiment of the present application;
FIG. 9 is a schematic diagram of oral disease information in an editable state according to an embodiment of the present application;
FIG. 10 is another schematic diagram of a visualization interface according to an embodiment of the present application;
FIG. 11 is another schematic diagram of a visualization interface according to an embodiment of the present application;
FIG. 12 is another schematic diagram of a visualization interface according to an embodiment of the present application;
FIG. 13 is another schematic diagram of a visualization interface according to an embodiment of the present application;
FIG. 14 is a flowchart illustrating a method for generating an oral health examination report according to an embodiment of the present application;
FIG. 15 is another flowchart illustrating a method for generating an oral health examination report according to an embodiment of the present application;
FIG. 16 is an interaction flowchart between a client and a server according to an embodiment of the present application;
FIG. 17 is a schematic diagram illustrating a screenshot management method according to an embodiment of the present application;
FIG. 18 is a schematic diagram of a visualization interface according to an embodiment of the present application;
FIG. 19 is another schematic diagram of a visualization interface according to an embodiment of the present application;
FIG. 20 is another schematic diagram of a visualization interface according to an embodiment of the present application;
FIG. 21 is another schematic diagram of a visualization interface according to an embodiment of the present application;
FIG. 22 is a block diagram of an apparatus for displaying oral health examination information according to an embodiment of the present application;
FIG. 23 is a block diagram of an apparatus for generating an oral health examination report according to an embodiment of the present application;
FIG. 24 is a block diagram of a screenshot management apparatus according to an embodiment of the present application;
FIG. 25 is a schematic diagram of a hardware structure of a computer device according to an embodiment of the present application.

### DETAILED DESCRIPTION

Exemplary embodiments will be described in detail here, examples of which are illustrated in the accompanying drawings. When the following description refers to the drawings, the same numbers in different drawings denote the same or similar elements unless otherwise indicated. The implementations described in the following exemplary embodiments do not represent all implementations consistent with the present application. Instead, they are merely examples of apparatuses and methods consistent with some aspects of the present application as detailed in the appended claims.

The terminology used in the present application is for the purpose of describing particular embodiments only and is not intended to limit the present application. The singular forms "a", "an", and "the" used in the present application and the appended claims are also intended to include plural forms unless the context clearly indicates otherwise. It should also be understood that the term "and/or" as used herein refers to and encompasses any and all possible combinations of one or more of the associated listed items.

It should be understood that, although the terms "first", "second", "third", etc. may be used herein to describe various information, this information should not be limited by these terms. These terms are only used to distinguish information of the same type from one another. For example, without departing from the scope of the present application, first information may also be referred to as second information, and similarly, second information may also be referred to as first information. Depending on the context, the word "if" as used herein may be interpreted as "when", "upon", or "in response to determining".

Doctor-patient communication can help doctors better understand patient's needs and expectations, and can also help patients better understand treatment plans and expected outcomes.

In related technologies, when doctors communicate the condition with patients after performing an oral examination, due to patients' lack of certain professional knowledge, they have difficulty understanding oral health examination information through the doctor's verbal description. Therefore, the present application proposes a method for displaying oral health examination information.

Generating an oral health examination report after performing an oral health examination for a patient is an important medical service provided by medical institutions. The oral health examination report usually records the patient's basic information and condition information. Doctors can use it to track and manage the patient's condition, better understand the patient's condition and treatment effectiveness, in order to further develop more effective treatment plans.

In related technologies, an oral health examination report is generated based on a fixed oral health examination report template. Names of various oral diseases displayed on this oral health examination report are all generic names. However, the same oral disease may have different customary names in different regions or countries, leading to increased difficulty for users in medical institutions in different regions to read and understand oral health examination reports displaying generic names, reducing work and communication efficiency accordingly, and failing to meet the customized needs of medical institutions in different regions.

The inventors have found through research that fixed oral health examination report templates define disease names for each oral disease as generic names. Therefore, the inventors thought of providing a plurality of oral health examination report templates that define different custom names for the same disease, enabling the generated oral health examination report to display custom names for oral diseases, thereby meeting the reading habits of medical institutions in different regions.

In some scenarios, when users communicate based on two-dimensional images, one party may lack corresponding professional knowledge, making it difficult to understand the content explaining the two-dimensional images, resulting in low communication efficiency. For example, during doctor-patient communication, doctors communicate with patients using paper-based medical images. However, due to patients' lack of certain professional knowledge, they have difficulty understanding the doctor's explanation of the medical images; engineers communicate with clients using paper-based design drawings. However, due to clients' lack of certain professional knowledge, they have difficulty understanding the engineer's explanation of the design drawings; teachers teach using pictures of cultural relics, artworks, or buildings. However, due to students' lack of certain professional knowledge, they have difficulty understanding the teacher's explanation of the pictures.

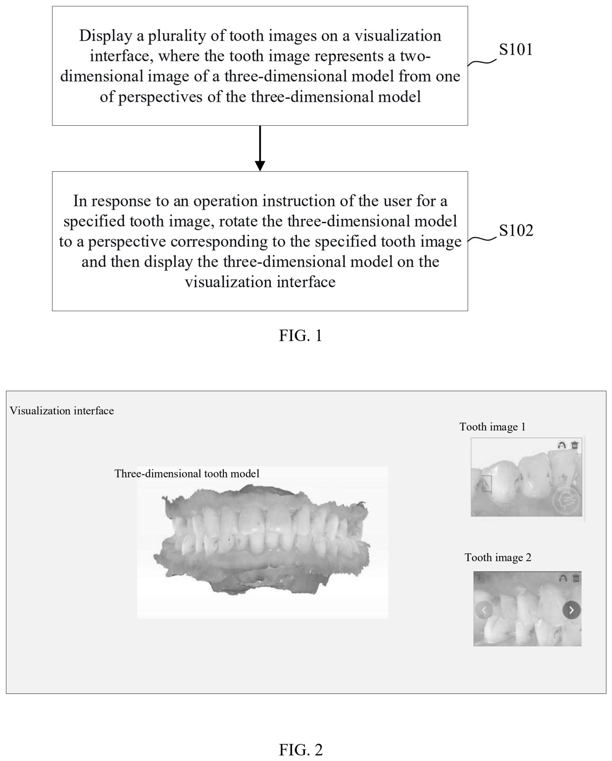

FIG. 1 is a flowchart illustrating a method for displaying oral health examination information according to an embodiment of the present application, including:
Step S101, a plurality of tooth images are displayed on a visualization interface, where the tooth image represents a two-dimensional image of a three-dimensional model from one of perspectives of the three-dimensional model.

In this embodiment, the three-dimensional model can be obtained by scanning with a three-dimensional scanning device such as an intraoral scanner, an extraoral scanner, a facial scanner, and a professional scanner. The three-dimensional model can be obtained by real-time scanning of a person's upper row of teeth and/or lower row of teeth by the scanning device, or can be a three-dimensional tooth model corresponding to the upper row of teeth and/or lower row of teeth sent by other devices. The present application does not limit the source of the three-dimensional model. The three-dimensional model can include a three-dimensional tooth model, a three-dimensional facial model (human face model), a human body model or another organ model, an industrial product model, an industrial equipment model, a cultural relic model, an artwork model, a prosthetic model, a medical instrument model, and an architectural model, etc. The present application does not limit this. The tooth images can include at least any one of the following: screenshots of the three-dimensional tooth model, tooth texture images, tooth near-infrared images, tooth X-ray images, CT (Computed Tomography) images, oral CBCT (Cone beam Computer Tomography) images, oral photographs, facial photographs, facial texture images, and screenshots of the three-dimensional facial model, etc. The present application does not limit this.

The tooth images can be collected by different medical devices, imported into a same server, and then synchronized to one or more clients by the server after performing operations such as screening, classification, analysis, and preprocessing, or directly without operations, for display on the visualization interface. This allows users to quickly understand data collected by different medical devices without switching devices, facilitating rapid and accurate analysis of oral health examination information from different angles.

In this embodiment, screenshots of the three-dimensional tooth model can be manual screenshots or screenshots determined by a deep learning method for recognizing the three-dimensional tooth model that include suspected lesion areas.

Screenshots of the three-dimensional facial model can be manual screenshots or screenshots determined by a deep learning method for recognizing the three-dimensional facial model that include suspected lesion areas; where the three-dimensional facial model can include tooth information. The three-dimensional facial model can be obtained by a facial scanner, or can be obtained by stitching a facial model obtained by a facial scanner and a tooth model obtained by an oral scanner.

Tooth texture images and facial texture images can be obtained by cameras of three-dimensional scanners. The tooth texture images and facial texture images can be fused with the three-dimensional tooth model and the three-dimensional facial model respectively. For example, when texturing the three-dimensional tooth model and the three-dimensional facial model, the tooth texture image and the facial texture image can be mapped onto the surfaces of the three-dimensional tooth model and the three-dimensional facial model respectively, thereby increasing the visual detail and realism of the three-dimensional tooth model and the three-dimensional facial model, generating high-definition three-dimensional tooth models and three-dimensional facial models.

Oral photographs (intraoral photographs) and facial photographs can be high-resolution photographs taken by high-definition cameras such as digital cameras. The oral photographs and facial photographs can be fused with the three-dimensional tooth model and the three-dimensional facial model. For example, texturing the three-dimensional tooth model, mapping oral photographs onto the surface of the three-dimensional tooth model, or mapping facial photographs onto the surface of the three-dimensional facial model, thereby further increasing the visual detail and realism of the three-dimensional tooth model and the three-dimensional facial model, generating even higher definition three-dimensional tooth models and three-dimensional facial models. For example: facial photographs can include multi-directional head photographs of the patient with teeth in occlusal and non-occlusal states when the mouth is open, and head photographs of the patient with the mouth closed. Oral photographs can be multi-directional close-up photographs of the mouth when the patient's mouth is open with teeth in occlusal and non-occlusal states.

Tooth near-infrared images can be obtained by near-infrared imagers; tooth X-ray images can be obtained by X-ray equipment; CT images can be obtained by CT scanners and can include information such as tooth crowns, tooth roots, mandible, and skull; oral CBCT images can be obtained by CBCT scanners and can include information such as tooth crowns, tooth roots, and mandible; the present application does not limit this.

Step S102, in response to an operation instruction of the user for a specified tooth image, the three-dimensional model is rotated to a perspective corresponding to the specified tooth image and then display the three-dimensional model on the visualization interface; where the specified tooth image includes a tooth image displaying a suspected lesion area, and the perspective corresponding to the specified tooth image is determined by pose parameters of the three-dimensional model bound to the tooth image.

In this embodiment, the three-dimensional model can be pre-displayed on the visualization interface, showing the user the process of model rotation, thereby enhancing the user's interactive visual experience.

In this embodiment, the pose parameters of the three-dimensional model can include an angle parameter, a position parameter, and a scale parameter. The angle parameter can be represented by a quaternion, a Euler angle, or a rotation vector, etc., to determine an attitude of the three-dimensional model in three-dimensional space; the position parameter can be represented by three-dimensional coordinates to determine a position of the three-dimensional model in three-dimensional space; the scale parameter can be represented by scaling factors. The scaling factors can include scaling ratios of the three-dimensional model in directions of an X-axis, a Y-axis, and a Z-axis of a three-dimensional coordinate system, used to shrink or enlarge the three-dimensional model.

In this embodiment, the three-dimensional model can also be enlarged or shrunk to a scale corresponding to the specified tooth image, and/or the tooth model can be moved to a position corresponding to the specified tooth image. The scale corresponding to the tooth image can be determined according to the scale parameter of the three-dimensional model bound to the tooth image. The position corresponding to the tooth image can be determined according to the position parameter of the three-dimensional model bound to the tooth image.

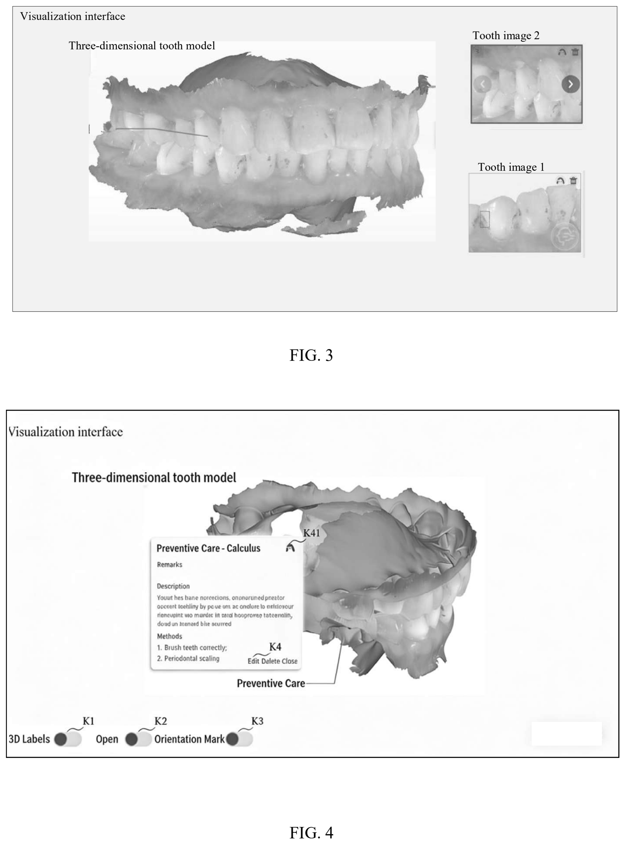

Exemplarily, as shown in FIG. 2 and FIG. 3, a three-dimensional tooth model, a tooth image 1, and a tooth image 2 are displayed on the visualization interface. FIG. 2 shows the three-dimensional tooth model in an initial pose. In response to the user's operation instruction for the tooth image 2, the three-dimensional tooth model in the initial pose is rotated to a perspective corresponding to tooth image 2, and the three-dimensional tooth model is enlarged according to the scale parameter of the three-dimensional tooth model bound to the tooth image 2, the three-dimensional tooth model with changed pose is shown as FIG. 3. Furthermore, a position of a center point of the tooth image 2 on the three-dimensional tooth model can be marked to highlight the suspected lesion area in the tooth image 2.

The user referred to in the present application can include doctors and patients. This embodiment, by responding to the user's operation instruction for a tooth image displaying a suspected lesion area, displays on the visualization interface the three-dimensional model rotated to the perspective corresponding to the tooth image, enabling the patient to directly see the position of the suspected lesion area on the three-dimensional model from the visualization interface, thereby allowing the patient to intuitively understand the oral health examination information through the visualization interface.

In one embodiment, as shown in FIG. 4, at least one control can be displayed on the visualization interface. The controls can include:
A first control K1, for enabling the user to trigger an operation to display a plurality of disease labels on the three-dimensional model (e.g., the three-dimensional tooth model in FIG. 4); where the plurality of disease labels are used to indicate a plurality of suspected lesion areas on the three-dimensional model, and information of the disease label represents an oral disease name corresponding to the suspected lesion area; or,
A second control K2, for enabling the user to trigger an operation to switch between display states of the three-dimensional model; the display states include an open state and a closed state; or,
A third control K3, for enabling the user to trigger an operation to display a plurality of orientation labels on the three-dimensional model; where the plurality of orientation labels are used to indicate a plurality of tooth areas on the three-dimensional model, and information of the orientation label represents a name of the tooth area; or,
A fourth control K4, for enabling the user to trigger an operation to edit oral disease information corresponding to a suspected lesion area; where a sub-control K41 included in the fourth control is used to trigger an operation to update tooth position number information of the suspected lesion area; the tooth number represents a position of a tooth in the three-dimensional model.

In the present application, controls can be buttons, sliders, or any other form of controls. The controls can be located anywhere on the visualization interface, and descriptive information can be added to the controls for user identification. The present application does not limit this.

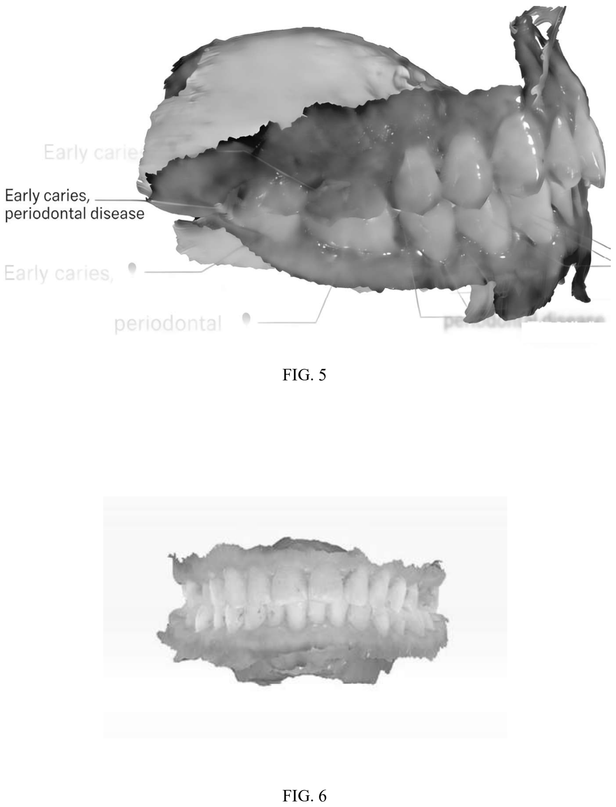

Exemplarily, as shown in FIG. 5, after the user triggers the first control K1, a plurality of disease labels on the three-dimensional tooth model can be displayed. Each disease label indicates the position of its corresponding suspected lesion area on the three-dimensional tooth model, and the information of the disease label represents the oral disease name corresponding to that suspected lesion area, enabling the patient to intuitively see the position of the suspected lesion area on the three-dimensional tooth model and the corresponding oral disease name through the visualization interface, facilitating doctor-patient communication about the condition.

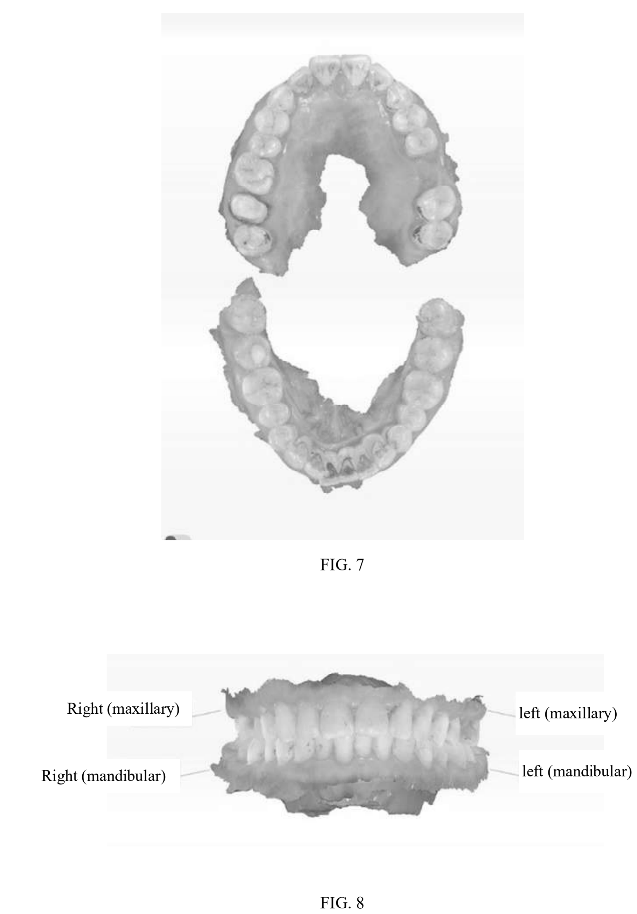

Exemplarily, as shown in FIG. 6 and FIG. 7, after the user triggers the second control K2, the display state of the three-dimensional tooth model can be switched to the closed state as shown in FIG. 6, or the open state as shown in FIG. 7.

Exemplarily, as shown in FIG. 8, after the user triggers the third control K3, a plurality of orientation labels on the three-dimensional tooth model can be displayed. Each orientation label indicates a different tooth area on the three-dimensional tooth model, and the information of the orientation label represents the name of its corresponding tooth area. The tooth areas can include a left maxillary area, a right maxillary area, a left mandibular area, and a right mandibular area, etc. The present application does not limit this.

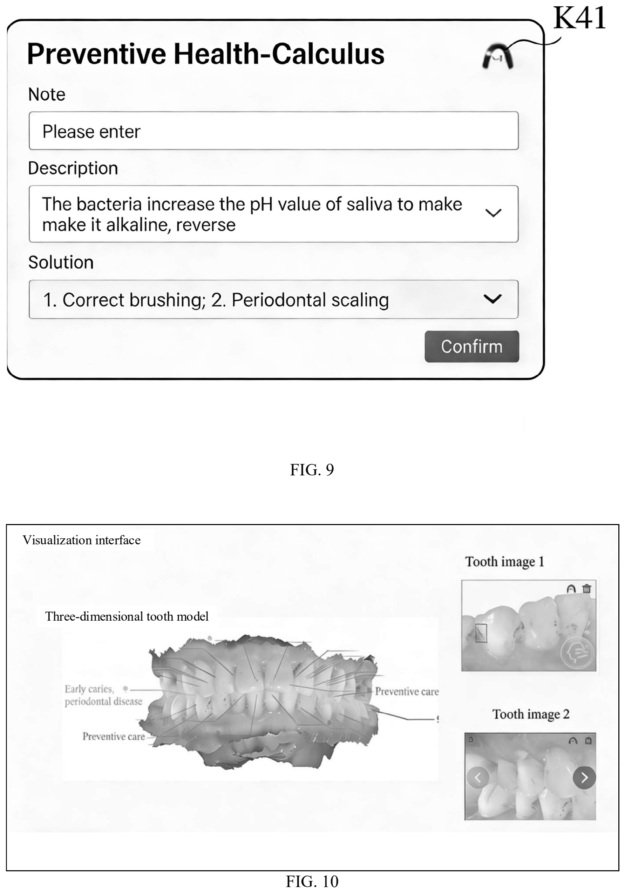

Exemplarily, as shown in FIG. 9, after the user triggers the fourth control K4, the oral disease information corresponding to a suspected lesion area can be edited. After triggering the sub-control K41 included in the fourth control, the tooth position number information of the suspected lesion area can be updated.

In one embodiment, the method for displaying oral health examination information can further include:
Step S103, in response to a user's rotation operation on the three-dimensional model, pose of labels on the three-dimensional model are adjusted according to a change amount of pose parameters during the rotation of the three-dimensional model, so that relative positions between the labels and the three-dimensional model remain consistent; where the labels at least include disease labels and orientation labels.

Exemplarily, when the user rotates the three-dimensional model, a rigid body transformation matrix used during the rotation of the three-dimensional model can be synchronously applied to the labels on the three-dimensional model to update the coordinates of the labels, adjust the pose of the labels, so that during a rotation process, the relative positions between the labels and the three-dimensional model remain consistent, and the positions on the three-dimensional model indicated by the labels remain unchanged.

In one embodiment, the method for displaying oral health examination information can further include:
Step S104, after the rotation operation on the three-dimensional model is completed, if a label occluded by the three-dimensional model is determined based on a current set of coordinate points of the three-dimensional model and a current set of coordinate points of the labels, a opacity of the label is reduced, and/or the pose of the label is adjusted.

In this embodiment, when a label is occluded by the three-dimensional model, the area indicated by that label may not be fully displayed in the three-dimensional model from the current perspective. At this time, the opacity of the label can be reduced, thereby highlighting the labels corresponding to the areas displayed in the three-dimensional model from the current perspective, enhancing the user's visual experience. Moreover, when a label is occluded by the three-dimensional model, the pose of the label can also be adjusted, such as adjusting a size, a position, or a rotation angle of the label, thereby avoiding problems where labels are occluded or multiple labels overlap, making it difficult for users to view and trigger the labels. It can also make the labels face the user, facilitating user viewing.

In one embodiment, disease labels can be pre-bound to tooth images displaying suspected lesion areas. The method for displaying oral health examination information can further include:
Step S105, in response to an operation instruction of the user for a disease label, oral disease information corresponding to the suspected lesion area is displayed, and/or the tooth image bound to the disease label is selected, and a display order of the tooth image bound to the disease label is ranked before other displayed tooth images.

The oral disease information can include at least any one of the following: an oral disease name, tooth position number information of the suspected lesion area, a symptom description, a treatment plan, and an educational video, etc. The present application does not limit this.

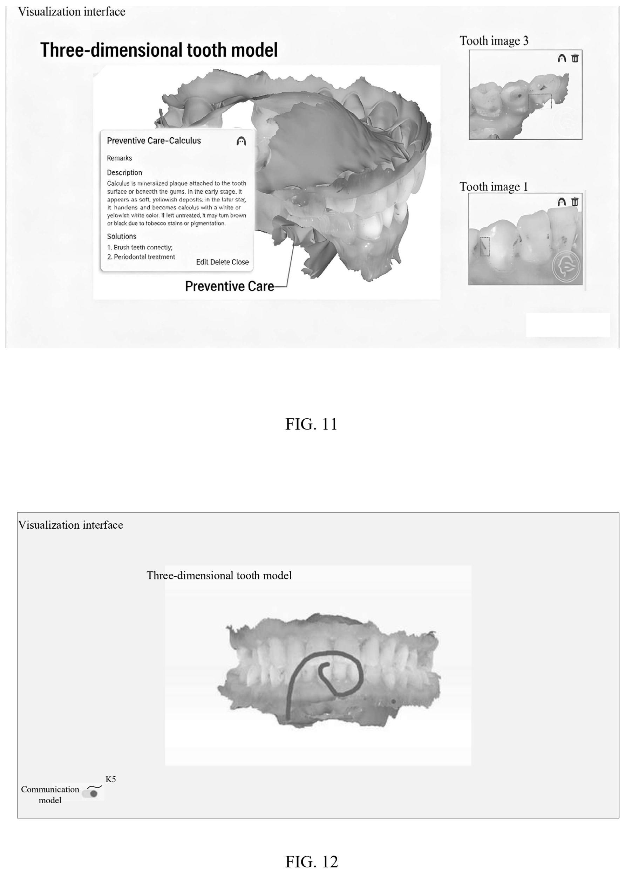

Exemplarily, as shown in FIG. 10 and FIG. 11, in response to an operation instruction of the user for a specified label among a plurality of labels on the three-dimensional tooth model, the tooth image 3 bound to this specified label can be selected and its display order can be ranked before other tooth images. Additionally, the oral disease information corresponding to the suspected lesion area displayed in tooth image 3 bound to this specified label can be displayed on the visualization interface.

In one embodiment, as shown in FIG. 12, a fifth control K5 can be displayed on the visualization interface to switch to a mode that allows the user to make marks on the three-dimensional model (e.g., the three-dimensional tooth model in FIG. 12); the specified tooth image can further include a tooth image displaying marks; the method for displaying oral health examination information can further include:
Step S106, in a case where the user has marked the three-dimensional model, in response to a user's rotation operation on the three-dimensional model, a screenshot of the marked three-dimensional model is taken before rotation and the screenshot is saved as a tooth image displaying marks; where the tooth image displaying marks is bound with the pose parameters and mark information of the marked three-dimensional model before rotation.

In this embodiment, when performing step S102 in response to the user's operation instruction for the specified tooth image, it can further include:
Step S1021, the specified tooth image is determined to be a tooth image displaying mark information.

Step S1022, rotating the three-dimensional model to the perspective corresponding to the specified tooth image and then displaying the three-dimensional model on the visualization interface can further include: displaying the mark information on the visualization interface.

In this embodiment, in a case where the user has marked the three-dimensional model, after responding to the user's rotation operation on the three-dimensional model, the marks made by the user on the three-dimensional model before rotation can also be cleared to facilitate the user's next marking operation.

In one embodiment, the suspected lesion area and the oral disease name corresponding to the suspected lesion area can be obtained based on a result of recognizing the three-dimensional model by a pre-trained oral detection model, and/or based on a result of recognizing the tooth image by a user.

In one embodiment, the step of obtaining the suspected lesion area and the oral disease name corresponding to the suspected lesion area based on the result of recognizing the three-dimensional model by the pre-trained oral detection model can include:
Identifying a plurality of suspected lesion areas of the three-dimensional model based on the pre-trained oral detection model, and obtaining a disease confidence set corresponding to several oral diseases for each suspected lesion area;
For each suspected lesion area, based on the disease confidence set, or based on the disease confidence set and an oral disease classification order preset in a target oral examination report template, determining a candidate oral disease list corresponding to the suspected lesion area;

Determining the oral disease name corresponding to the suspected lesion area from the candidate oral disease list according to a preset rule and/or in response to a user's selection operation.

In this embodiment, the oral detection model can include various machine learning models for identifying oral diseases such as a periodontal disease recognition model, a caries recognition model, and a tooth width measurement model. The present application does not limit this.

The target oral examination report template can be obtained from a server based on a template ID in response to a template acquisition instruction carrying the template ID. The server can be configured to provide an oral examination report template set. The oral examination report template set includes a plurality of oral examination report templates generated for the same oral disease. The oral examination report template defines candidate names for an oral disease, and the candidate names are generic names or custom names.

In the present application, the server can include a cloud server, a local server, etc. The present application does not limit this. In some embodiments, the method for displaying oral health examination information can run on a client. The client can include a software client, a mobile client, and a web client, etc. In some embodiments, there are multiple clients and one server. Any client receives editing information for oral health examination information from a user and sends the editing information to the server. The server receives the editing information and synchronizes the editing information to all clients.

In this embodiment, the oral disease name corresponding to the suspected lesion area can be determined from the candidate oral disease list according to a preset rule and/or in response to a user's selection operation. Then, the candidate name corresponding to the oral disease defined in the target oral examination report template can be used as the oral disease name corresponding to a currently identified suspected lesion area. The preset rule can be defaulting to selecting the Nth candidate oral disease in the candidate oral disease list; the user's selection operation can refer to the user directly selecting a certain candidate oral disease in the candidate oral disease list, or can refer to the user selecting a specified rule. The specified rule can be automatically selecting the first candidate oral disease in the candidate oral disease list corresponding to each suspected lesion area.

In one embodiment, the step of determining the candidate oral disease list corresponding to the suspected lesion area based on the disease confidence set can include:
From the disease confidence set, determining several oral diseases corresponding to the top N highest disease confidences as elements of the candidate oral disease list; where N is a positive integer.

Specifically, for example, if the oral diseases corresponding to the top 3 highest confidences in the disease confidence set are severe dental crowding, anterior crossbite, and anterior deep overjet respectively, then the aforementioned oral diseases can be used as elements of the candidate oral disease list, i.e., candidate oral diseases.

In one embodiment, the step of determining the candidate oral disease list corresponding to the suspected lesion area based on the disease confidence set and the oral disease classification order preset in the target oral examination report template can include:
From the disease confidence set, determining several oral diseases corresponding to the top N highest disease confidences as elements of the candidate oral disease list; where N is a positive integer and is less than a preset length of the candidate oral disease list;
Based on the oral disease classification order preset in the target oral examination report template, determining several oral diseases of the same category corresponding to a first candidate oral disease as elements of the candidate oral disease list; where the first candidate oral disease is the oral disease corresponding to the Nth highest disease confidence in the disease confidence set.

In this embodiment, in addition to obtaining candidate oral diseases corresponding to the suspected lesion area based on confidence, several oral diseases of the same category as the oral disease corresponding to the Nth highest confidence can also be selected based on the oral disease classification order preset in the target oral examination report template, as elements of the candidate oral disease list, i.e., candidate oral diseases. Oral diseases of the same category can refer to oral diseases belonging to the same level as the first candidate oral disease, or can refer to other oral diseases at the upper M level (M is a positive integer) corresponding to the level of the first candidate oral disease. The present application does not limit this.

Specifically, for example, if the preset length of the candidate oral disease list is 3, and the oral disease corresponding to the highest confidence determined from the disease confidence set is severe dental crowding, it is used as an element of the candidate oral disease list. At this time, if the number of elements in the candidate oral disease list is less than 3, then further, based on the oral disease classification order preset in the target oral examination report template, it is determined that severe dental crowding belongs under the classification of malocclusion. At this time, oral diseases at the same level can be preferentially selected, for example, anterior crossbite and anterior deep overjet, as elements of the candidate oral disease list so that the number of elements in the candidate oral disease list meets its preset length requirement. If the number of oral diseases at the same level is still insufficient, then other oral diseases can be searched level by level upwards as elements in the candidate oral disease list until the number of elements in the candidate oral disease list meets its preset length requirement.

In this embodiment, a preset oral disease list can also be determined based on the oral disease classification order preset in the target oral examination report template. In response to a user's selection operation, an oral disease can be determined from the preset oral disease list as an element of the candidate oral disease list.

In one embodiment, the step of obtaining oral health examination information based on the result of recognizing the tooth image by the user can include:
Determining a plurality of suspected lesion areas annotated by the user on the tooth image, and determining the oral disease name corresponding to each suspected lesion area according to an oral disease selected by the user from a preset oral disease list; where the preset oral disease list is determined according to the oral disease classification order preset in the target oral examination report template.

In one embodiment, the method for displaying oral health examination information can further include:
Step S107, multi-source data is classified and displayed on the visualization interface; where the multi-source data is imported by the user and/or obtained from a server. The categories of the multi-source data include at least any one of the following:
Three-dimensional model, screenshots of the three-dimensional model, texture images, oral photographs, facial photographs, near-infrared images, X-ray images, CT (Computed Tomography) images, and CBCT (Cone beam Computer Tomography) images.

The multi-source data can be collected by different medical devices, imported into the same server, and then synchronized to one or more clients by the server for display on the visualization interface. This allows users to quickly understand data collected by different medical devices without switching devices, facilitating rapid and accurate analysis of health examination information from different angles.

In this embodiment, the screenshots of the three-dimensional model can be manual screenshots or screenshots determined by a deep learning method for recognizing the three-dimensional model that include suspected lesion areas. The three-dimensional facial model can include tooth information. The three-dimensional facial model can be obtained by a facial scanner, or can be obtained by stitching a facial model obtained by a facial scanner and a tooth model obtained by an oral scanner.

The texture images can be obtained by cameras of three-dimensional scanners, including tooth texture images, facial texture images, etc. Texture images can be fused with the three-dimensional model. For example, when texturing the three-dimensional model, the texture image can be mapped onto the surface of the three-dimensional model, thereby increasing the visual detail and realism of the three-dimensional model and generating a high-definition three-dimensional model.

The oral photographs (intraoral photographs) and facial photographs can be high-resolution photographs taken by high-definition cameras such as digital cameras. They can be fused with the three-dimensional model. For example, texturing the three-dimensional model, mapping intraoral photographs and/or facial two-dimensional images onto the surface of the three-dimensional model, thereby further increasing the visual detail and realism of the three-dimensional model and generating an even higher definition three-dimensional model. For example: facial photographs can include multi-directional head photographs of the patient with teeth in occlusal and non-occlusal states when the mouth is open, and head photographs of the patient with the mouth closed. Oral photographs can include multi-directional close-up photographs of the mouth when the patient's mouth is open with teeth in occlusal and non-occlusal states.

The near-infrared images can be obtained by near-infrared imagers, including tooth near-infrared images, facial near-infrared images, etc.; the X-ray images can be obtained by X-ray equipment, including tooth X-ray images, facial X-ray images, etc.; the CT images can be obtained by CT scanners and can include information such as tooth crowns, tooth roots, mandible, and skull; the CBCT images can be obtained by CBCT scanners and can include information such as tooth crowns, tooth roots, and mandible; the present application does not limit this.

In some embodiments, the classification of multi-source data can be performed automatically by a computer according to preset rules, or in response to user selection for classification. The automatic classification method can include one or more of the following: a clustering method, or a deep learning algorithm (e.g., an image classification model based on a neural network), or classification by filename semantic recognition, or classification by format.

In one embodiment, the multi-source data can first undergo a primary classification based on a format of the multi-source data. Three-dimensional model can be in a format such as STL (STereoLithography), OBJ, and PLY (Polygon File Format); the screenshots of the three-dimensional model, the oral photographs, the texture images, the near-infrared images, the X-ray images, and the facial photographs can be in formats such as JPG, PNG; the CT images and the CBCT images can be in DICOM (Digital Imaging and Communications in Medicine) format. Then, a secondary classification can be performed using a neural network-based image classification model. For example, files of the same format can be classified according to preset rules such as file content, whether the file is black and white, etc., to make a classification result more accurate. The present application does not limit this.

In some other embodiments, a semantic recognition can be performed on a filename of the multi-source data to obtain keyword information, and the multi-source data can be classified accordingly. For example, if the filename includes "intraoral photo", it can be determined that the category of this multi-source data is oral photograph. In addition, the keyword information such as patient name and acquisition time can also be recognized from the filenames of the multi-source data, which can be used to further classify the multi-source data.

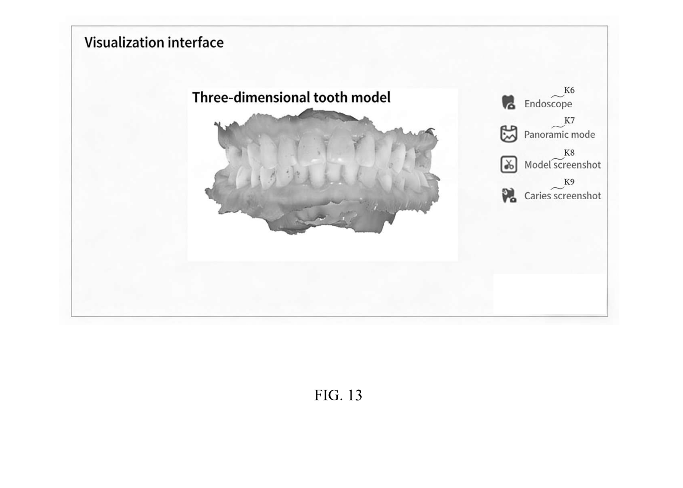

Exemplarily, as shown in FIG. 13, a sixth control K6 can be displayed on the visualization interface. In response to a user's operation on the sixth control K6, an endoscopic screenshot of teeth can be imported; a seventh control K7 can also be displayed on the visualization interface. In response to a user's operation on the seventh control K7, a panoramic radiograph of teeth can be imported; an eighth control K8 can also be displayed on the visualization interface. In response to a user's operation on the eighth control K8, a screenshot of the currently displayed three-dimensional tooth model can be taken and saved; a ninth control K9 can also be displayed on the visualization interface. In response to a user's operation on the ninth control K9, a caries screenshot of teeth can be imported.

In one embodiment, an oral examination report can be generated to present oral health examination information to the user. The oral examination report can display basic information such as a patient name, an age, a gender, a doctor's name, a contact phone number, and a consultation date; it can also display a tooth position diagram marking the tooth positions corresponding to suspected lesion areas, and the categories of oral diseases corresponding to the suspected lesion areas; it can also display five views of the three-dimensional model, including a front view, a left side view, a right side view, a maxillary view, and a mandibular view; it can also display detailed information for each suspected lesion area, including images corresponding to the suspected lesion area and oral disease information, etc. The oral examination report can also display an associated QR code, allowing users to conveniently view the content of the corresponding oral examination report by scanning the QR code. Furthermore, the oral examination report can be presented in a format such as PDF, an image, or as an HTML5 page. On the HTML5 page, the oral examination report can also display the three-dimensional model in open state, closed state, showing only the maxilla, and showing only the mandible, facilitating user rotation of the model to view the oral condition.

In the present application, operation instructions can be triggered by the user through clicking, touching, sliding, etc., on operation objects such as tooth images, controls, and labels on the visualization interface, or can be voice commands and gestures input by the user. The present application does not limit this.

In the present application, the three-dimensional model, tooth images, controls, etc., can be displayed anywhere on the visualization interface. The present application does not limit this.

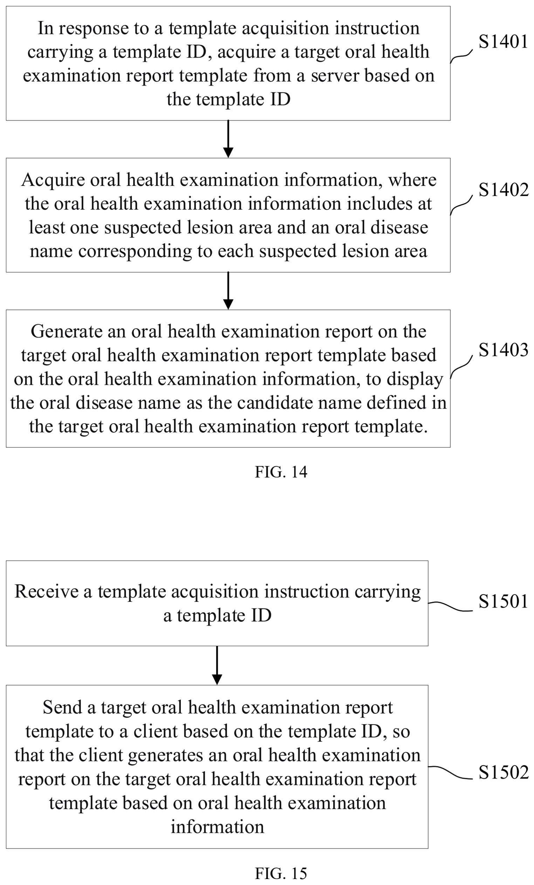

The method for displaying oral health examination information further includes generating an oral health examination report. FIG. 14 is a flowchart illustrating a method for generating an oral health examination report according to an embodiment of the present application, including:
Step S1401, in response to a template acquisition instruction carrying a template ID, a target oral health examination report template is acquired from a server based on the template ID. The server is configured to provide an oral health examination report template set. The oral health examination report template set includes a plurality of oral health examination report templates generated for a same oral disease. The oral health examination report template defines a candidate name for the oral disease, and the candidate name is a generic name or a custom name.
Step S102, oral health examination information is acquired, where the oral health examination information includes at least one suspected lesion area and an oral disease name corresponding to each suspected lesion area.
Step S103, an oral health examination report on the target oral health examination report template is generated based on the oral health examination information, to display the oral disease name as the candidate name defined in the target oral health examination report template.

In this embodiment, the suspected lesion areas can be displayed in a form of two-dimensional images of the three-dimensional model from different angles or three-dimensional images of the three-dimensional model. The present application does not limit this.

In one embodiment, the server stores an oral disease dictionary. The oral disease dictionary can include oral disease IDs and generic names of oral diseases corresponding to the oral disease IDs, where each oral disease ID corresponds to one oral disease. An administrator can create the generic oral health examination report template on the server based on the oral disease dictionary. The generic oral health examination report template defines the candidate name for the oral disease as the generic name. Users from various medical institutions can pre-create custom oral health examination report templates on the server based on the oral disease dictionary. The name of each oral disease in the template defaults to the generic name. When creating a custom oral health examination report template, the user can create a corresponding custom name for a specified oral disease based on the oral disease ID. Then, the custom oral health examination report template defines the candidate name for the oral disease as the generic name or the custom name.

The technical solution of this embodiment, by acquiring the target oral health examination report template from the oral health examination report template set provided by the server when generating the examination report, and generating the oral health examination report on the target oral health examination report template based on the acquired oral health examination information, because the oral health examination report template set includes the plurality of oral health examination report templates that define different custom names for the same disease, enables the generated oral health examination report to display the custom name for the oral disease, thereby meeting reading habits of medical institutions in different regions, corresponding to different regional languages and medical terminology, improving work and communication efficiency, and satisfying the customized needs of medical institutions in different regions.

In one embodiment, the oral health examination information can be obtained based on a result of recognizing the three-dimensional model by a pre-trained oral detection model, and/or based on a result of recognizing the tooth image by a user.

The technical solution of this embodiment, by acquiring the result of recognizing the three-dimensional tooth model by the pre-trained oral detection model and results of recognizing different types of tooth images by the user to obtain oral health examination information, provides multiple data sources for the content of the oral health examination report, thereby making the generated oral health examination report more comprehensive.

In one embodiment, the oral health examination report template can further define oral disease information bound to the candidate name; where the oral disease information can include a symptom description, a treatment plan, an educational video, etc. The present application does not limit this.

In this embodiment, the step of generating the oral health examination report on the target oral health examination report template based on the oral health examination information can include:
Determining the candidate name corresponding to the oral disease name in the target oral health examination report template, and acquiring the oral disease information bound to the candidate name;
Generating an oral health examination report on the target oral health examination report template based on at least one suspected lesion area, the oral disease name corresponding to each suspected lesion area, and the oral disease information.

In this embodiment, when an administrator or a user from a medical institution creates a custom oral health examination report template on the server based on the oral disease dictionary, in addition to creating the candidate name corresponding to each oral disease, they can also create oral disease information bound to the candidate name. The oral disease information can include generic oral disease information and custom oral disease information.

The technical solution of this embodiment, by generating the oral health examination report based on the oral health examination information and the oral disease information bound to the candidate name, makes the oral health examination report more comprehensive and meets the customized needs of medical institutions in different regions.

In one embodiment, the method for generating the oral health examination report can further include:
Step S1404, the oral health examination report is uploaded to the server, so that the user can perform desensitization processing on the patient information in the oral health examination report on the server, analyze the oral health examination information in the oral health examination report, and/or train the oral detection model based on the oral health examination information.

In this embodiment, the desensitization processing can involve operations such as deletion, encryption, or replacement of sensitive data in the patient information, making the patient's identity undeterminable and protecting the patient's privacy. The present application does not limit this.

In this embodiment, analyzing the oral health examination information in the oral health examination report can specifically be: analyzing a plurality of oral health examination information stored in the server, statistically analyzing the oral disease names in the plurality of oral health examination information to obtain oral health status of residents in different regions, in order to provide corresponding operational guidance to medical institutions. For example, if the incidence of caries in a certain region is high, it may be recommended that medical institutions in that region focus on publicity for caries prevention and treatment.

In this embodiment, the user can use the oral health examination information stored in the server after desensitization processing for training the oral detection model, improving the accuracy of the oral detection model.

In the present application, clients can include software clients, mobile clients, web clients, etc. Servers can include cloud servers, local servers, etc. The present application does not limit this.

In one embodiment, the user can manage oral health examination information on the server, including: the user can edit oral health examination information on the server, generate new oral health examination reports on the server, where the user can edit the same oral health examination information on different types of servers; the user can distribute oral health examination information stored on the server to mini-programs, allowing users to generate oral health examination reports within the mini-program; the user can archive oral health examination information stored on the server by patient dimension to better track and manage the oral health status of each patient.

In another embodiment, the user can edit the same oral health examination information on various different types of clients, send the edited oral health examination information to the server, which synchronizes it and then sends it to the various different types of clients.

Specifically, there are multiple clients and one server. Any client receives editing information for oral health examination information from a user and sends the editing information to the server. The server receives the editing information and synchronizes it to all clients.

In one embodiment, the method for generating the oral health examination report can further include:
Step S1405, a preview version of the oral health examination report on the target oral health examination report template is generated based on the oral health examination information, so that the user can confirm an accuracy of the oral health examination information before generating a final oral health examination report.

In one embodiment, the oral health examination report can include a QR code associated with it, allowing the user to conveniently view the content of the corresponding oral health examination report by scanning the QR code. The oral health examination report can be in the format such as PDF or image. The present application does not limit this.

FIG. 15 is another flowchart illustrating a method for generating an oral health examination report according to an embodiment of the present application, including:
Step S1501, a template acquisition instruction carrying a template ID is received.
Step S1502, a target oral health examination report template is sent to a client based on the template ID, so that the client generates an oral health examination report on the target oral health examination report template based on oral health examination information.

The target oral health examination report template belongs to an oral health examination report template set. The oral health examination report template set includes a plurality of oral health examination report templates generated for the same oral disease. The oral health examination report template defines the candidate name for the oral disease, and the candidate name is a generic name or a custom name. The oral disease name is displayed in the oral health examination report as the candidate name defined in the target oral health examination report template.

In one embodiment, the oral health examination report template can further define oral disease information bound to the candidate names.

In this embodiment, the step of generating the oral health examination report on the target oral health examination report template based on the oral health examination information can include:
Determining the candidate name corresponding to the oral disease name in the target oral health examination report template, and acquiring the oral disease information bound to the candidate name;
Generating the oral health examination report on the target oral health examination report template based on the at least one suspected lesion area, the oral disease name corresponding to each suspected lesion area, and the oral disease information.

In one embodiment, the method for generating the oral health examination report can further include: receiving the oral health examination report uploaded by the client to the server, performing desensitization processing on the patient information in the oral health examination report, analyzing the oral health examination information in the oral health examination report, and/or training the oral detection model based on the oral health examination information.

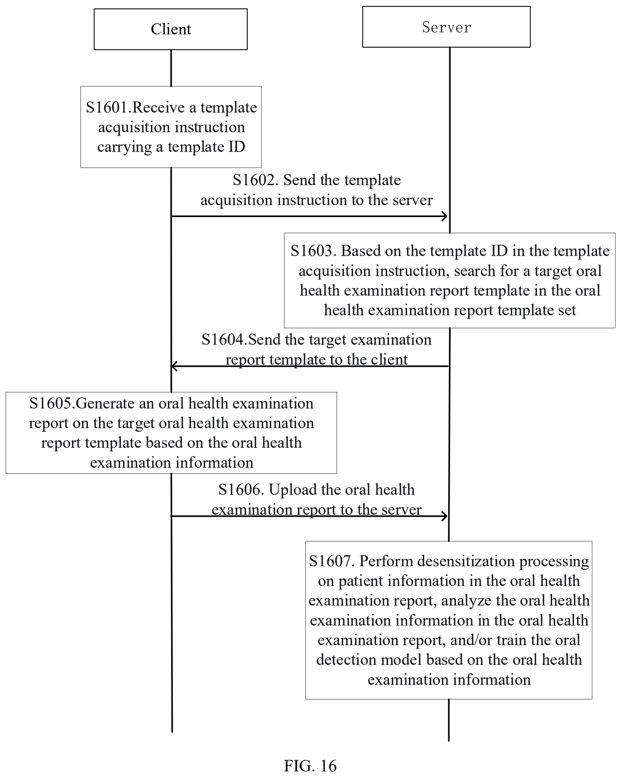

FIG. 16 is an interaction flowchart between a client and a server according to an embodiment of the present application. Exemplarily, as shown in FIG. 16, the interaction process between the client and the server can be:
Step S1601: The client receives a template acquisition instruction carrying a template ID.
Step S1602: The client sends the template acquisition instruction to the server.
Step S1603: The server, based on the template ID in the template acquisition instruction, searches for a target oral health examination report template in the oral health examination report template set.
Step S1604: The server sends the target examination report template to the client.
Step S1605: The client generates an oral health examination report on the target oral health examination report template based on the oral health examination information.
Step S1606: The client uploads the oral health examination report to the server.
Step S1607: The server performs desensitization processing on patient information in the oral health examination report, analyzes the oral health examination information in the oral health examination report, and/or trains the oral detection model based on the oral health examination information.

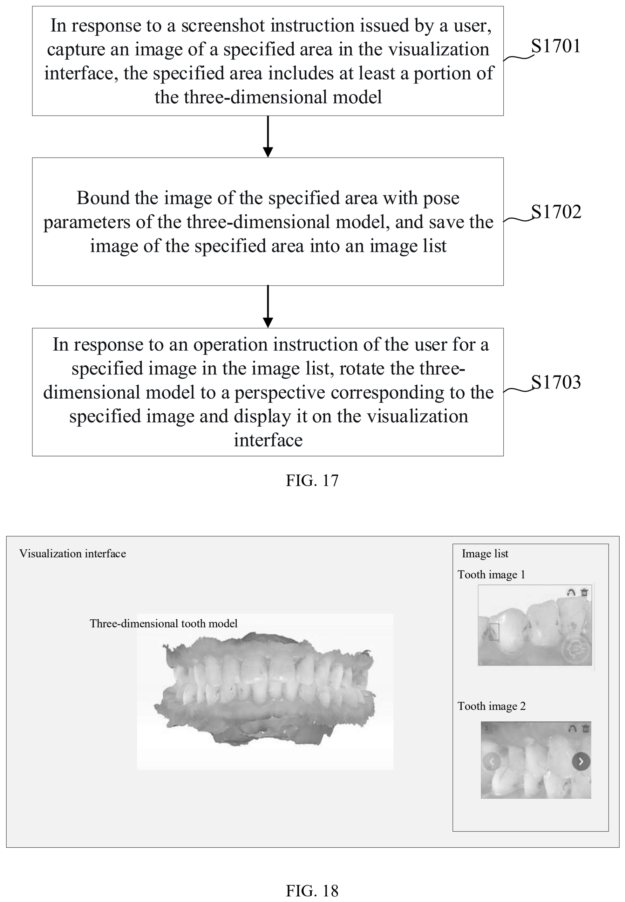

The method for displaying oral health examination information further includes screenshot management. FIG. 17 is a flowchart illustrating a screenshot management method according to an embodiment of the present application, including:
Step S1701: In response to a screenshot instruction issued by a user, an image of a specified area in the visualization interface is captured, the specified area including at least a portion of the three-dimensional model.
Step S1702: The image of the specified area is bound with pose parameters of the three-dimensional model, and the image of the specified area is saved into an image list; the image list includes a plurality of images, and the image of the specified area represents a two-dimensional image of the three-dimensional model from one of perspectives of the three-dimensional model.
Step S1703: In response to an operation instruction of the user for a specified image in the image list, the three-dimensional model is rotated to a perspective corresponding to the specified image and then is displayed on the visualization interface; the perspective corresponding to the specified image is determined by pose parameters of the three-dimensional model bound to the image.

In this embodiment, the screenshot instruction issued by the user can refer to an instruction issued when the user triggers a control for taking a screenshot on the visualization interface, or can refer to a voice instruction, a gesture instruction, or a remote control instruction issued by the user for indicating a screenshot. The present application does not limit the specific manner in which the user issues the screenshot instruction or the specific form of the screenshot instruction.

In this embodiment, the specified area can be the entire visualization interface displaying the three-dimensional model, or a region corresponding to a default display position of the three-dimensional model in the visualization interface, or a region selected by the user on the visualization interface targeting the three-dimensional model. The present application does not limit this.

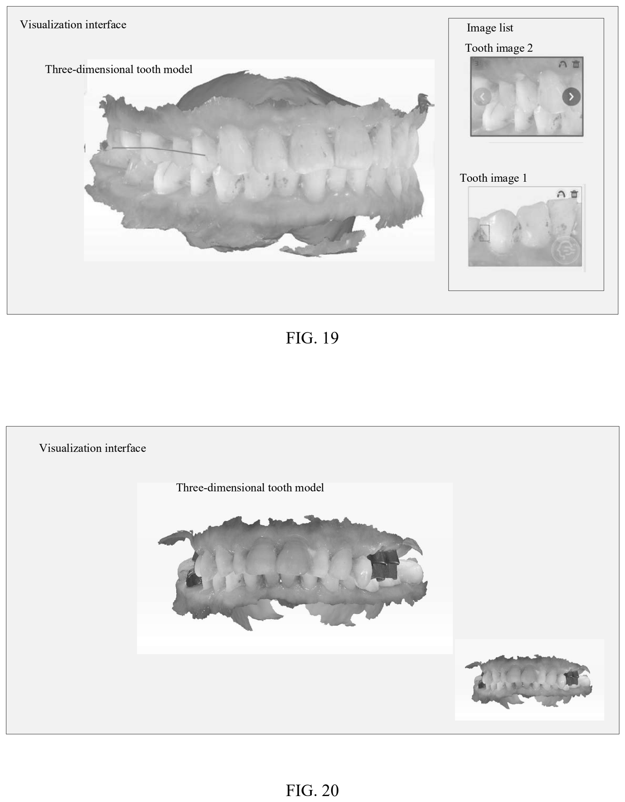

Exemplarily, as shown in FIG. 18 and FIG. 19, a three-dimensional tooth model and an image list including an image 1 and an image 2 are displayed on the visualization interface. FIG. 18 shows the three-dimensional tooth model in an initial pose. In response to the user's operation instruction for the image 2, the three-dimensional tooth model in the initial pose is rotated to the perspective corresponding to the image 2, and the three-dimensional tooth model is enlarged according to the scale parameter of the three-dimensional tooth model bound to the image 2, displaying the three-dimensional tooth model with a changed pose as shown in FIG. 19. Furthermore, a position of a center point of the image 2 on the three-dimensional tooth model can be marked to highlight the suspected tooth lesion area in the image 2.

This embodiment, by binding the captured image including at least a portion of the three-dimensional model with the pose parameters of the three-dimensional model and saving it into an image list, can then respond to an operation instruction of the user for a specified image in the image list and display on the visualization interface the three-dimensional model rotated to the perspective corresponding to the specified image, enabling the user to intuitively communicate based on the three-dimensional model displayed on the visualization interface, making it easier to understand highly professional content and improving communication efficiency.

In one embodiment, users can communicate regarding the three-dimensional model displayed on the visualization interface and record the communication content to generate a communication record. A type of the communication record can be a text record, an audio record, and a video record, etc. The present application does not limit this.

In this embodiment, a semantic recognition can be performed on the communication record to obtain a semantic recognition result. Based on the semantic recognition result, a communication summary can be generated. The communication summary can include summary information, communication time, participants, and a speaking record of each participant, etc. The summary information can include several keywords. Through the keywords, the positions of areas of interest to the user that exist on the three-dimensional model can be determined. At this time, screenshots can be taken for the areas of interest in the three-dimensional model and saved into the image list.

In this embodiment, based on the semantic recognition result, description information of an area of interest can also be determined through the keywords. Based on the image displaying the area of interest and the corresponding description information of the area of interest, an examination report can be generated. For different types of three-dimensional models, the keywords extracted from the semantic recognition result will vary.

For example, for a three-dimensional tooth model, keywords can be "tooth number 1 has a problem, there is caries", "tooth number 5 is fine", etc. Through the keywords, it can be determined which tooth area corresponding to which tooth position number on the three-dimensional tooth model is the area of interest. The description information of the area of interest can include a health status of the tooth corresponding to the tooth position number. An area of interest with health problems can be called a suspected lesion area. The description information of the area of interest can also include the oral disease name corresponding to the suspected lesion area. The type of generated examination report can be an oral health examination report. If there is a corresponding near-infrared image for the image of the area of interest, the image of the area of interest and the near-infrared image can be associated and used together to generate the oral health examination report.

For another example, for a three-dimensional facial model, keywords can be "the nasal tip point position is low", "there are blackheads on the nose", "the eye distance is too wide", etc. Through the keywords, it can be determined which facial feature area on the three-dimensional facial model is the area of interest. The description information of the area of interest can include problems existing in the facial feature area. The type of generated examination report can be a facial examination report.

For another example, for an industrial product model, keywords can be "the center hole size of the gear is too small", "there is wear on the top of the central shaft", etc. Through the keywords, it can be determined which component area on the industrial product model is the area of interest. The description information of the area of interest can include problems existing in the component area. The type of generated examination report can be an industrial product inspection report.

In this embodiment, the semantic recognition can be based on a natural language processing model, such as GPT (Generative Pre-trained Transformers), OPT (Open Pre-trained Transformers), and LLaMA (Large Language Model Meta Al), etc. For other types of three-dimensional models, corresponding examination reports can also be generated. The present application does not limit this.

In one embodiment, the screenshot management method can further include:
Step S1703: In response to an operation instruction of the user for the captured image, at least one of the following controls is displayed on the visualization interface:
An eleventh control K11, for enabling the user to trigger an operation for label management on the captured image;
A twelfth control K12, for enabling the user to trigger an operation to switch to a mode that allows the user to make a mark on the captured image. The twelfth control K12 includes at least one of the following sub-controls:
   A first sub-control K21, for enabling the user to trigger an operation to adjust a color of the mark;
   A second sub-control K22, for enabling the user to trigger an operation to adjust a width of the mark;
   A third sub-control K23, for enabling the user to trigger an operation to adjust a timeliness of the mark.

In this embodiment, the label management on the captured image can include operations such as generating a label on the captured image, editing the label, and deleting the label. The label can be a text label, a pattern label, etc. The present application does not limit this.

In this embodiment, the user can trigger the third sub-control to adjust the timeliness of the mark on the image. When a specified time (e.g., 3 seconds) is reached, the mark can be hidden or continue to be displayed.

In this embodiment, the visualization interface can also display a thirteenth control K13 (not shown in the figure), for enabling the user to trigger an operation to switch a display state of the image list. The visualization interface can also display an image switching control for the image list, for enabling the user to trigger an operation to switch the image currently displayed in the visualization interface within the image list.

In this embodiment, at least one of the following controls can also be displayed on the visualization interface:
A fourteenth control K14, for enabling the user to trigger an operation to switch to a mode that allows erasing the mark on the captured image;
A fifteenth control K15, for enabling the user to trigger an operation to undo a most recent operation;
A sixteenth control K16, for enabling the user to trigger an operation to redo a most recent undone operation;
A seventeenth control K17, for enabling the user to trigger an operation to hide the control displayed on the visualization interface after responding to the user's operation instruction for the captured image;
An eighteenth control K18, for enabling the user to trigger an operation to save the marked captured image to the image list.

In the present application, controls can be buttons, sliders, or any other form of controls. The controls can be located anywhere on the visualization interface, and descriptive information can be added to the controls for user identification. The present application does not limit this.

In one embodiment, after capturing the image of the specified area in the visualization interface, the screenshot management method can further include:
Step S1704: The captured image is shrunk and displayed in a preset area of the visualization interface;
The user's operation instruction for the captured image in step S1703 can be an operation instruction triggered by the user for the shrunken captured image within a predetermined time.

In this embodiment, the screenshot management method can further include:
Step S1705: When no operation instruction triggered by the user for the shrunken captured image is received within the predetermined time, and/or when an operation instruction triggered by the user for an area outside the shrunken captured image on the visualization interface is received, the shrunken captured image is hidden.

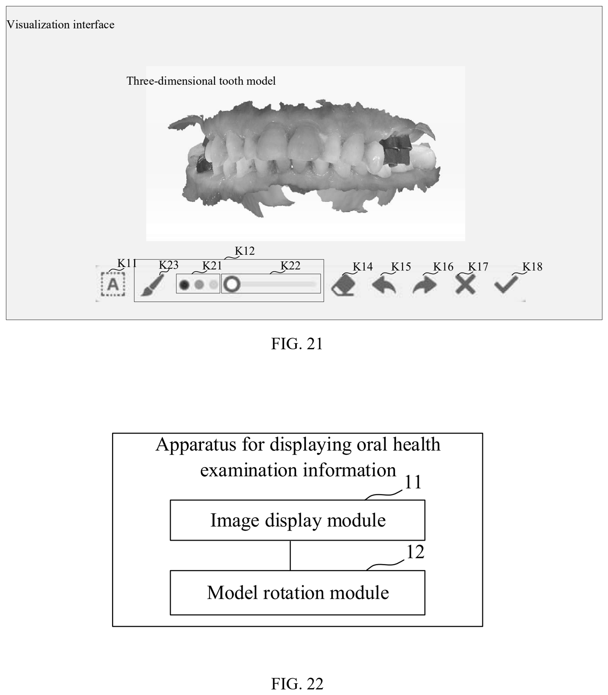

Exemplarily, as shown in FIG. 20, the captured image is shrunk and displayed in a lower right corner area of the visualization interface. If the user triggers this shrunken captured image within three seconds, as shown in FIG. 21, the aforementioned several controls are displayed on the visualization interface. Additionally, this shrunken captured image can be enlarged again and displayed at a specified position on the visualization interface, allowing the user to perform operations such as making marks and label management on this captured image.

In one embodiment, the user's operation instruction for the captured image in step S1703 can be an operation instruction triggered by the user for the captured image within the image list.

In one embodiment, the specified image can further include images displaying mark information. When performing step S1702 in response to the user's operation instruction for the specified image in the image list, it can further include:
Step S17021, the specified image is determined to be an image displaying mark information.

The step S1702, where rotating the three-dimensional model to the perspective corresponding to the specified image and then displaying the three-dimensional model on the visualization interface further includes:
Step S17022, the mark information is displayed on the visualization interface.

In one embodiment, the image list can also include images displaying mark information. In response to an operation instruction of the user for an image displaying mark information in the image list, that image displaying mark information can be displayed on the visualization interface.

In one embodiment, images in the image list can be filtered and only images with mark information can be displayed. The user can trigger an operation to switch the currently displayed image with mark information through an image switching control for the image list, improving communication efficiency.

In one embodiment, when the three-dimensional model is a three-dimensional tooth model or a three-dimensional facial model, the specified image can include an image displaying a suspected tooth lesion area., the suspected tooth lesion area and the oral disease name corresponding to the suspected tooth lesion area.

In this embodiment, when a least one suspected tooth lesion area of the three-dimensional model is identified based on a pre-trained oral detection model, the screenshot can be taken for the suspected tooth lesion area in the three-dimensional model and saved into the image list.

In one embodiment, the user can recognize the captured image. The step of obtaining the suspected tooth lesion area and the oral disease name corresponding to the suspected tooth lesion area based on the user's recognition result of the image can include: determining at least one suspected tooth lesion area annotated by the user on the image, and determining the oral disease name corresponding to each suspected tooth lesion area according to an oral disease selected by the user from a preset oral disease list; where the preset oral disease list is determined according to an oral disease classification order preset in a target oral health examination report template.

In this embodiment, the user can annotate the suspected tooth lesion area on the image by selecting the tooth position number corresponding to the suspected tooth lesion area.

FIG. 22 is a block diagram of an apparatus for displaying oral health examination information according to an embodiment of the present application, including: an image display module 11, configured to display a plurality of tooth images on a visualization interface, where the tooth image represents a two-dimensional image of a three-dimensional model from one of its perspectives; a model rotation module 12, configured to, in response to an operation instruction of the user for a specified tooth image, rotate the three-dimensional model to a perspective corresponding to the specified tooth image and display the three-dimensional model on the visualization interface; where the specified tooth image includes a tooth image displaying a suspected lesion area, and the perspective corresponding to the specified tooth image is determined by pose parameters of the three-dimensional model bound to the tooth image.

In one embodiment, the apparatus for displaying oral health examination information can further include: a first control display module 13, configured to display at least one control module on the visualization interface; the at least one control module includes: a first control module 1301, for enabling the user to trigger an operation to display a plurality of disease labels on the three-dimensional model; where the plurality of disease labels are used to indicate a plurality of suspected lesion areas on the three-dimensional model, and the information of the disease label represents the oral disease name corresponding to the suspected lesion area; or, a second control module 1302, for enabling the user to trigger an operation to switch between display states of the three-dimensional model; the display states include an open state and a closed state; or, a third control module 1303, for enabling the user to trigger an operation to display a plurality of orientation labels on the three-dimensional model; where the plurality of orientation labels are used to indicate a plurality of tooth areas on the three-dimensional model, and the information of the orientation label represents the name of the tooth area; or, a fourth control module 1304, for enabling the user to trigger an operation to edit oral disease information corresponding to a suspected lesion area; where a sub-control module 13041 included in the fourth control module is used to trigger an operation to update tooth position number information of the suspected lesion area; the tooth number represents a position of a tooth in the three-dimensional model.

In one embodiment, the apparatus for displaying oral health examination information can further include: a rotation synchronization module 14, configured to, in response to a user's rotation operation on the three-dimensional model, adjust the pose of labels on the three-dimensional model according to a change amount of pose parameters during a rotation of the three-dimensional model, so that relative positions between the labels and the three-dimensional model remain consistent; where the labels at least include disease labels and orientation labels.

In one embodiment, the apparatus for displaying oral health examination information can further include: a label adjustment module 15, configured to, after the rotation operation on the three-dimensional model is completed, if it is determined based on the coordinate point set of the three-dimensional model and the coordinate point set of the labels that a label is occluded by the three-dimensional model, then reduce an opacity of the label, and/or adjust the pose of the label.

In one embodiment, disease the labels can be pre-bound to tooth images displaying suspected lesion areas. The apparatus for displaying oral health examination information can further include: a first display module 16, configured to, in response to an operation instruction of the user for a disease label, display oral disease information corresponding to the suspected lesion area, and/or a second display module 17, configured to select the tooth image bound to the disease label, and rank the display order of the tooth image bound to the disease label before other displayed tooth images; where the oral disease information includes at least any one of the following: the oral disease name, tooth position number information of the suspected lesion area, the symptom description, the treatment plan, and the educational video.

In one embodiment, the specified tooth image can further include tooth images displaying marks. The apparatus for displaying oral health examination information can further include: a second control display module 18, configured to display a fifth control on the visualization interface to switch to a mode that allows the user to make a mark on the three-dimensional model; a model screenshot module 19, configured to, in a case where the user has marked the three-dimensional model, in response to a user's rotation operation on the three-dimensional model, take a screenshot of the marked three-dimensional model before rotation and save it as a tooth image displaying the mark; where the tooth image displaying the mark is bound with the pose parameters and mark information of the marked three-dimensional model before rotation; the model rotation module 12 is further configured to, when responding to the user's operation instruction for the specified tooth image, determine that the specified tooth image is a tooth image displaying mark information; rotating the three-dimensional model to the perspective corresponding to the specified tooth image and then displaying the three-dimensional model on the visualization interface further includes: displaying the mark information on the visualization interface.

In one embodiment, the suspected lesion area and the oral disease name corresponding to the suspected lesion area can be obtained based on a result of recognizing the three-dimensional model by a pre-trained oral detection model, and/or based on a result of recognizing the tooth image by a user.

In one embodiment, the apparatus for displaying oral health examination information can further include: an image classification display module 20, configured to classify multi-source data and display multi-source data on the visualization interface; where the multi-source data is imported by the user and/or obtained from a server. The categories of the multi-source data include at least any one of the following:
Three-dimensional model, screenshots of the three-dimensional model, texture images, oral photographs, facial photographs, near-infrared images, X-ray images, CT (Computed Tomography) images, and CBCT (Cone beam Computer Tomography) images.

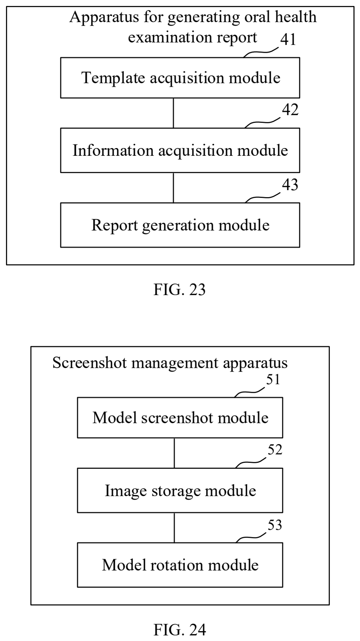

The apparatus for displaying oral health examination information further includes an apparatus for generating an oral health examination report. FIG. 23 is a block diagram of an apparatus for generating an oral health examination report according to an embodiment of the present application, including: a template acquisition module 41, configured to, in response to a template acquisition instruction carrying a template ID, acquire a target oral health examination report template from a server based on the template ID, the server being configured to provide an oral health examination report template set, the oral health examination report template set including a plurality of oral health examination report templates generated for a same oral disease, the oral health examination report template defining a candidate name for the oral disease, the candidate name being a generic name or a custom name; an information acquisition module 42, configured to acquire oral health examination information, where the oral health examination information includes at least one lesion area and an oral disease name corresponding to each suspected lesion area; a report generation module 43, configured to generate an oral health examination report on the target oral health examination report template based on the oral health examination information, to display the oral disease name as the candidate name defined in the target oral health examination report template.

The apparatus for displaying oral health examination information further includes a screenshot management apparatus. FIG. 24 is a block diagram of a screenshot management apparatus according to an embodiment of the present application, including: a model screenshot module 51, configured to, in response to a screenshot instruction issued by a user, capture an image of a specified area in the visualization interface, the specified area including at least a portion of a three-dimensional model; an image storage module 52, configured to bind the image with pose parameters of the three-dimensional model, and save the image into an image list; the image list includes a plurality of images, and the image represents a two-dimensional image of the three-dimensional model from one of perspectives of the three-dimensional model; a model rotation module 53, configured to, in response to an operation instruction of the user for a specified image in the image list, rotate the three-dimensional model to a perspective corresponding to the specified image and then display the three-dimensional model on the visualization interface; the perspective corresponding to the specified image is determined by pose parameters of the three-dimensional model bound to the image.

In one embodiment, the specified image can include several images displaying areas of interest. The capture timing for the image displaying the area of interest can include, a moment when the area of interest exists in the three-dimensional model is determined based on a semantic recognition result of a communication record regarding the three-dimensional model.

In one embodiment, the screenshot management apparatus can further include: a semantic recognition module 54, configured to, based on the semantic recognition result, determine description information of the area of interest; a report generation module 55, configured to generate an examination report based on the image displaying the area of interest and the corresponding description information.

In one embodiment, the screenshot management apparatus can further include: a control display module, configured to, in response to an operation instruction of the user for the captured image, display at least one of the following controls on the visualization interface: an eleventh control K11, for enabling the user to trigger an operation for label management on the captured image; a twelfth control K12, for enabling the user to trigger an operation to switch to a mode that allows the user to make marks on the captured image.

The twelfth control K12 includes at least one of the following sub-controls: a first sub-control K21, for enabling the user to trigger an operation to adjust a color of a mark; a second sub-control K22, for enabling the user to trigger an operation to adjust a width of the mark; a third sub-control K23, for enabling the user to trigger an operation to adjust a timeliness of the mark.

In one embodiment, the screenshot management apparatus can further include: an image adjustment module 56, configured to shrink the captured image and display captured image in a preset area of the visualization interface; the user's operation instruction for the captured image in the model rotation module 53 can be an operation instruction triggered by the user for the shrunken captured image within a predetermined time.

In one embodiment, the specified image can further include images displaying mark information. The model rotation module 53 can also be used to determine that the specified image is an image displaying mark information and display the mark information on the visualization interface.

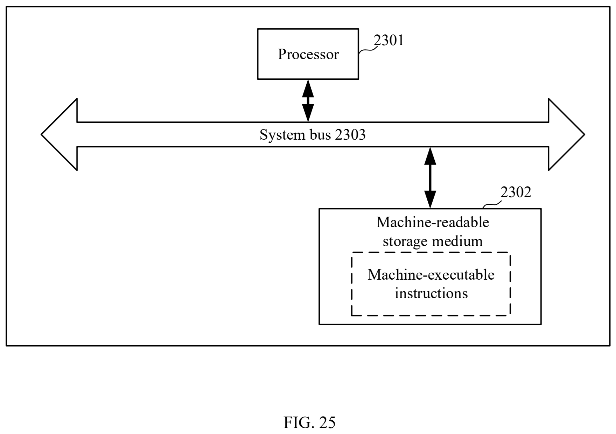

FIG. 25 is a schematic diagram of a hardware structure of a computer device according to an embodiment of the present application. The computer device can include a processor 2301 and a machine-readable storage medium 2302 storing machine-executable instructions. The processor 2301 can communicate with the machine-readable storage medium 2302 via a system bus 2303. Moreover, by reading and executing the machine-executable instructions corresponding to the oral health examination information display logic in the machine-readable storage medium 2302, the processor 2301 can execute the oral health examination information display method described above.

The machine-readable storage medium 2302 mentioned here can be any electronic, magnetic, optical, or other physical storage device that can contain or store information, such as executable instructions, data, etc. For example, the machine-readable storage medium 2302 can include at least one of the following storage media: volatile memory, non-volatile memory, and other types of storage media. The volatile memory can be a RAM (Random Access Memory), and the non-volatile memory can be flash memory, storage drives (such as hard disk drives), solid-state drives, storage disks (such as optical discs, DVDs, etc.).

Based on the method of any of the above embodiments, an embodiment of the present application also provides a computer-readable storage medium. The medium stores a computer program. When the computer program is executed by a processor, it can be used to execute the oral health examination information display method of any of the above embodiments.

The systems, apparatuses, modules, or units illustrated in the above embodiments can be implemented by computer chips or entities, or by products with certain functions. A typical implementation device is a computer. The specific form of the computer can be a personal computer, laptop computer, cellular phone, camera phone, smartphone, personal digital assistant, media player, navigation device, email device, game console, tablet computer, wearable device, or a combination of any of these devices.

It should also be noted that the terms "include", "comprise", or any other variation thereof are intended to cover non-exclusive inclusion, so that a process, method, article, or device that includes a series of elements includes not only those elements, but also other elements not explicitly listed, or elements inherent to such process, method, article, or device. Without further limitation, an element defined by the phrase "including a..." does not preclude the existence of additional identical elements in the process, method, article, or device that includes the element.

Specific embodiments of the present application have been described above. Other embodiments fall within the scope of the appended claims. In some cases, the actions or steps recited in the claims can be performed in an order different from that in the embodiments and still achieve desirable results. In addition, the processes depicted in the drawings do not necessarily require the specific order or sequential order shown to achieve desirable results. In certain implementations, multitasking and parallel processing are also possible or may be advantageous.

The descriptions of "specific examples" or "some examples" mean that the specific features, structures, materials, or characteristics described in connection with the embodiments or examples are included in at least one embodiment or example of the present application. In the present application, schematic references to the above terms do not necessarily refer to the same embodiment or example. Moreover, the described specific features, structures, materials, or characteristics can be combined in any suitable manner in any one or more embodiments or examples.

Those skilled in the art will readily think of other implementations of the present application after considering the specification and practicing the application disclosed herein. The present application is intended to cover any variations, uses, or adaptive changes of the present application. These variations, uses, or adaptive changes follow the general principles of the present application and include common general knowledge or conventional technical means in the technical field not claimed in the present application. The specification and examples are considered exemplary only, with the true scope and spirit of the application being indicated by the following claims.

It should be understood that the present application is not limited to the precise structure that has been described above and shown in the drawings, and that various modifications and changes can be made without departing from its scope. The scope of the present application is limited only by the appended claims.

The above are only preferred embodiments of the present application and are not intended to limit the present application. Any modifications, equivalent replacements, improvements, etc., made within the spirit and principles of the present application shall be included within the protection scope of the present application.

### INDUSTRIAL APPLICABILITY

The method for displaying oral health examination information provided in the present disclosure, by responding to an operation instruction of the user for a tooth image displaying a suspected lesion area, displays on the visualization interface the three-dimensional model rotated to the perspective corresponding to the tooth image, enabling the patient to directly see the position of the suspected lesion area on the three-dimensional model from the visualization interface, thereby allowing the patient to intuitively understand the oral health examination information through the visualization interface. It has strong industrial applicability.

## Claims

1. A method for displaying oral health examination information, **characterized in that** the method comprises:
displaying a plurality of tooth images on a visualization interface, the tooth image representing a two-dimensional image of a three-dimensional model from one of perspectives of the three-dimensional model;
in response to an operation instruction of a user for a specified tooth image, rotating the three-dimensional model to a perspective corresponding to the specified tooth image and displaying the three-dimensional model on the visualization interface; wherein the specified tooth image comprises a tooth image displaying a suspected lesion area,
and the perspective corresponding to the specified tooth image is determined by pose parameters of the three-dimensional model bound to the tooth image.

2. The method according to claim 1, wherein at least one control is displayed on the visualization interface, and the at least one control comprises:
a first control, for enabling the user to trigger an operation to display a plurality of disease labels on the three-dimensional model; wherein the plurality of disease labels are configured to indicate a plurality of suspected lesion areas on the three-dimensional model, and information of the disease label represents an oral disease name corresponding to the suspected lesion area; or,
a second control, for enabling the user to trigger an operation to switch between display states of the three-dimensional model; the display states comprising an open state and a closed state; or,
a third control, for enabling the user to trigger an operation to display a plurality of orientation labels on the three-dimensional model; wherein the plurality of orientation labels are configured to indicate a plurality of tooth areas on the three-dimensional model, and information of the orientation label represents a name of a tooth area; or,
a fourth control, for enabling the user to trigger an operation to edit oral disease information corresponding to the suspected lesion area; wherein a sub-control comprised in the fourth control is configured to trigger an operation to update tooth position number information of the suspected lesion area; a tooth number represents a position of a tooth in the three-dimensional model.

3. The method according to claim 2, further comprising:
in response to a user's rotation operation on the three-dimensional model, adjusting a pose of labels on the three-dimensional model according to a change amount of pose parameters during a rotation of the three-dimensional model, so that relative positions between the labels and the three-dimensional model remain consistent; wherein the labels at least comprise disease labels and orientation labels;
after the rotation operation on the three-dimensional model is completed, in response that a label is occluded by the three-dimensional model determined based on a current coordinate point set of the three-dimensional model and a current coordinate point set of the labels, reducing an opacity of the label, and/or adjusting a pose of the label.

4. The method according to claim 2, wherein the disease labels are pre-bound to tooth images displaying suspected lesion areas, and the method further comprises:
in response to an operation instruction of the user for a disease label, displaying oral disease information corresponding to the suspected lesion area, and/or selecting the tooth image bound to the disease label, and ranking a display order of the tooth image bound to the disease label before other displayed tooth images;
wherein the oral disease information comprises at least any one of: an oral disease name, tooth position number information of the suspected lesion area, a symptom description, a treatment plan, and an educational video.

5. The method according to claim 1, wherein a fifth control is displayed on the visualization interface to switch to a mode that allows the user to make a mark on the three-dimensional model; the specified tooth image further comprises a tooth image displaying the mark; the method further comprises:
under a condition that the user has marked the three-dimensional model, in response to a user's rotation operation on the three-dimensional model, taking a screenshot of the marked three-dimensional model before rotation and saving the screenshot as a tooth image displaying the mark; wherein the tooth image displaying the mark is bound with pose parameters and mark information of the marked three-dimensional model before rotation;
when responding to the user's operation instruction for the specified tooth image, the method further comprises:
determining that the specified tooth image is a tooth image displaying mark information;
rotating the three-dimensional model to the perspective corresponding to the specified tooth image and displaying the three-dimensional model on the visualization interface further comprises:
displaying the mark information on the visualization interface.

6. The method according to claim 1, further comprising:
classifying multi-source data and displaying the multi-source data on the visualization interface; wherein the multi-source data is imported by the user and/or obtained from a server, and categories of the multi-source data comprise at least any one of:
the three-dimensional model, screenshots of the three-dimensional model, texture images, oral photographs, facial photographs, near-infrared images, X-ray images, computed tomography (CT) images, and cone beam computer tomography (CBCT) images.

7. The method according to claim 1, applied to a client, further comprising:
in response to a template acquisition instruction carrying a template ID;
acquiring a target oral health examination report template from a server based on the template ID, the server being configured to provide an oral health examination report template set, the oral health examination report template set comprising a plurality of oral health examination report templates generated for a same oral disease, the oral health examination report template defining a candidate name for an oral disease, the candidate name being a generic name or a custom name;
acquiring oral health examination information, the oral health examination information being obtained based on a result of recognizing the three-dimensional model by a pre-trained oral detection model, and/or based on a result of recognizing the tooth image by a user, the oral health examination information comprising at least one suspected lesion area and an oral disease name corresponding to each suspected lesion area;
generating an oral health examination report on the target oral health examination report template based on the oral health examination information, to display the oral disease name as the candidate name defined in the target oral health examination report template.

8. The method according to claim 7, wherein acquiring the oral health examination information based on the result of recognizing the three-dimensional model by the pre-trained oral detection model comprises: identifying, based on the pre-trained oral detection model, at least one lesion area of the three-dimensional model, and obtaining a confidence set corresponding to several oral diseases for each suspected lesion area; for each suspected lesion area, based on the disease confidence set, or based on the disease confidence set and an oral disease classification order preset in the target oral health examination report template, determining a candidate oral disease list corresponding to the suspected lesion area; determining the oral disease name corresponding to the suspected lesion area from the candidate oral disease list according to a preset rule and/or in response to a user's selection operation;
wherein acquiring the oral health examination information based on the result of recognizing the tooth image by the user comprises: determining at least one suspected lesion area annotated by the user on the tooth image, and determining the oral disease name corresponding to each suspected lesion area according to an oral disease selected by the user from a preset oral disease list; wherein the preset oral disease list is determined according to an oral disease classification order preset in the target oral health examination report template; the tooth image comprises at least any one of: a screenshot of a three-dimensional tooth model in the three-dimensional model, a tooth texture image, a tooth near-infrared image, a tooth X-ray image, a CT image, an oral CBCT image, an oral photograph, a facial photograph, a facial texture image, and a screenshot of a three-dimensional facial model in the three-dimensional model.

9. The method according to claim 8, wherein determining the candidate oral disease list corresponding to the suspected lesion area based on the disease confidence set and the oral disease classification order preset in the target oral health examination report template comprises: from the disease confidence set, determining several oral diseases corresponding to top N highest disease confidences as elements of the candidate oral disease list; wherein N is a positive integer and is less than a preset length of the candidate oral disease list; based on the oral disease classification order preset in the target oral health examination report template, determining several oral diseases of a same category corresponding to a first candidate oral disease as elements of the candidate oral disease list; wherein the first candidate oral disease is the oral disease corresponding to the Nth highest disease confidence in the disease confidence set.

10. The method according to claim 7, wherein the oral health examination report template further defines oral disease information bound to the candidate name; generating the oral health examination report on the target oral health examination report template based on the oral health examination information comprises:
determining the candidate name corresponding to the oral disease name in the target oral health examination report template, and acquiring the oral disease information bound to the candidate name;
generating the oral health examination report on the target oral health examination report template based on the at least one suspected lesion area, the oral disease name corresponding to each suspected lesion area, and the oral disease information.

11. The method according to claim 8, further comprising:
uploading the oral health examination report to the server, so that the user can perform a desensitization processing on patient information in the oral health examination report on the server, analyze the oral health examination information in the oral health examination report, and/or train the oral detection model based on the oral health examination information.

12. The method according to claim 1, applied to a server, further comprising:
receiving a template acquisition instruction carrying a template ID;
sending a target oral health examination report template to a client based on the template ID, so that the client generates an oral health examination report on the target oral health examination report template based on oral health examination information;
wherein the oral health examination information comprises at least one suspected lesion area and an oral disease name corresponding to each suspected lesion area; the target oral health examination report template belongs to an oral health examination report template set, the oral health examination report template set comprising a plurality of oral health examination report templates generated for a same oral disease, the oral health examination report template defining a candidate name for an oral disease, the candidate name being a generic name or a custom name; the oral disease name is displayed in the oral health examination report as the candidate name defined in the target oral health examination report template.

13. The method according to claim 12, wherein the oral health examination report template further defines oral disease information bound to the candidate names; generating the oral health examination report on the target oral health examination report template based on the oral health examination information comprises:
determining the candidate name corresponding to the oral disease name in the target oral health examination report template, and acquiring the oral disease information bound to the candidate name;
generating the oral health examination report on the target oral health examination report template based on the at least one suspected lesion area, the oral disease name corresponding to each suspected lesion area, and the oral disease information.

14. The method according to claim 12, wherein the oral health examination information is obtained based on a result of recognizing the three-dimensional model by a pre-trained oral detection model, and/or based on a result of recognizing the tooth image by a user; the method further comprises:
receiving the oral health examination report uploaded by the client, performing a desensitization processing on patient information in the oral health examination report, analyzing the oral health examination information in the oral health examination report, and/or training the oral detection model based on the oral health examination information.

15. The method according to claim 1, wherein before responding to the user's operation instruction for the specified tooth image, the method further comprises:
in response to a screenshot instruction issued by a user, capturing an image of a specified area in the visualization interface, the specified area comprising at least a portion of the three-dimensional model;
binding the image of the specified area with pose parameters of the three-dimensional model, and saving the image of the specified area into an image list; the image list comprises a plurality of images, and the image of the specified area represents a two-dimensional image of the three-dimensional model from one of perspectives of the three-dimensional model.

16. The method according to claim 15, wherein after capturing the image of the specified area in the visualization interface, the method further comprises:
shrinking the captured image and displaying the captured image in a preset area of the visualization interface;
wherein the operation instruction for the captured image is an operation instruction triggered by the user for the shrunken captured image within a predetermined time.

17. The method according to claim 15, wherein the specified tooth image comprises several images displaying areas of interest; a capture timing for the image displaying the area of interest comprises a moment when the area of interest exists in the three-dimensional model is determined based on a semantic recognition result of a communication record regarding the three-dimensional model.

18. The method according to claim 17, further comprising:
based on the semantic recognition result, determining description information of the area of interest;
based on the image displaying the area of interest and the corresponding description information of the area of interest, generating an examination report.

19. The method according to claim 15, further comprising:
in response to an operation instruction of the user for the captured image, displaying at least one of following controls on the visualization interface:
an eleventh control, for enabling the user to trigger an operation for a label management on the captured image;
a twelfth control, for enabling the user to trigger an operation to switch to a mode that allows the user to make a mark on the captured image;
a thirteenth control, for enabling the user to trigger an operation to switch a display state of the image list;
wherein the twelfth control comprises at least one of following sub-controls:
a first sub-control, for enabling the user to trigger an operation to adjust a color of the mark;
a second sub-control, for enabling the user to trigger an operation to adjust a width of the mark;
a third sub-control, for enabling the user to trigger an operation to adjust a timeliness of the mark.

20. An apparatus for displaying oral health examination information, **characterized in that** the apparatus comprises:
an image display module, configured to display a plurality of tooth images on a visualization interface, wherein the tooth image represents a two-dimensional image of a three-dimensional model from one of perspectives of the three-dimensional model;
a model rotation module, configured to, in response to an operation instruction of the user for a specified tooth image, rotate the three-dimensional model to a perspective corresponding to the specified tooth image and display the three-dimensional model on the visualization interface; wherein the specified tooth image comprises a tooth image displaying a suspected lesion area, and the perspective corresponding to the specified tooth image is determined by pose parameters of the three-dimensional model bound to the tooth image.

21. A computer device, **characterized in that** the computer device comprises a processor and a machine-readable storage medium, the machine-readable storage medium stores machine-executable instructions executable by the processor, and the processor executes the machine-executable instructions to implement the method according to any one of claims 1-19.

22. A computer-readable storage medium, **characterized in that** the medium stores a computer program, and the computer program, when executed by a processor, implements the method according to any one of claims 1-19.
